# EUROPEAN PATENT APPLICATION

(11) **EP 4 371 998 A1**
(43) Date of publication of application: **22.05.2024**
(21) Application number: 21953718.0
(22) Date of filing: 18.08.2021
(51) Int. Cl.: C07K 14/28, C12N 15/31, C12Q 1/6869, G01N 33/487

(54) **MUTANT OF PORIN MONOMER, PROTEIN PORE AND APPLICATION THEREOF**

(71) Applicant: Qitan Technology Ltd., Chengdu, Sichuan 610041 (CN)
(72) Inventor: LIU, Shaowei, Chengdu city, Sichuan 610041 (CN); HE, Jingxiong, Chengdu city, Sichuan 610041 (CN); LI, Qianwen, Chengdu city, Sichuan 610041 (CN); YUE, Feifei, Chengdu city, Sichuan 610041 (CN)
(74) Representative: Forresters IP LLP
(86) International application number: PCT/CN2021/113272
(87) International publication number: WO 2023/019471

(57) **Abstract**

The present invention belongs to the technical field of characterization of target analyte properties, and particularly provides a mutant of a porin monomer, a protein pore comprising same, and use thereof in the detection of a target analyte, wherein an amino acid of the mutant of the porin monomer comprises mutations at one or more of positions corresponding to A74, P75, G76, N77, A78, T79, N80, and F81 of SEQ ID NO: 1.

## Description

### TECHNICAL FIELD

The present invention belongs to the technical field of characterization of target analyte properties, and particularly relates to a mutant of a porin monomer, a protein pore comprising same, and use thereof in the detection of a target analyte.

### BACKGROUND

With the research on the structure and sequence of nucleic acids, nucleic acid sequencing technologies have been continuously developed, become the core field of life science research, and play a great driving role in the technical development in the fields of biology, chemistry, electricity, life science, medicine, and the like. It is one of the hot issues in the post-Human Genome Project to develop a novel, rapid, accurate, low-cost, high-precision, and high-throughput nucleic acid sequencing technology by using nanopores.

Nanopore sequencing technology, also known as the fourth generation sequencing technology, is a gene sequencing technology that uses a single-stranded nucleic acid molecule as a sequencing unit. It utilizes a nanopore capable of providing an ion current channel to enable the single-stranded nucleic acid molecule to pass through the nanopore driven by electrophoresis. When the nucleic acid passes through the nanopore, the current of the nanopore is reduced and different signals generated are able to be read in real time to acquire sequence information.

The nanopore sequencing is mainly characterized in that the reading length is very long, the accuracy rate is relatively high, and most error regions occur in homopolymeric oligonucleotide regions. The nanopore sequencing can not only realize natural DNA and RNA sequencing, but also directly acquire base modification information about DNA and RNA. For example, methylated cytosine can be directly read by the nanopore sequencing, and bisulfite treatment on genomes is not needed in advance like the second generation sequencing method, which greatly promotes the direct research on epigenetic phenomenon at the genome level. As a novel platform, the nanopore detection technology has the advantages of low cost, high throughput, no label, and the like.

Nanopore analysis technology originated from the invention of the Coulter counter and the single-channel current record technology. In 1976, Neher and Sakamann, the Nobel Prize winners in Physiology and Medicine, utilized patch clamp technology to measure membrane potential and research membrane proteins and ion channels, thereby promoting the practical application progress of the nanopore sequencing technology. In 1996, Kasianowicz et al. proposed a new idea for DNA sequencing by using α-hemolysin, which is a milestone marker of single molecule sequencing by biological nanopores. Subsequently, the research reports on the biological nanopores such as MspA porin, bacteriophage Phi29 connector, and the like enrich the research on the nanopore analysis technology. In 2001, Li et al. opened a new era of solid-state nanopore research. Limited by the development of the semiconductor and material industries, solid-state nanopore sequencing has progressed slowly.

One of the key points of the nanopore sequencing technology lies in the design of a special biological nanopore, in which a reading head structure formed in a constriction zone of a pore can cause the blockage of a pore channel current when a single-stranded nucleic acid (such as ssDNA) molecule passes through the nanopore, thereby transiently affecting the intensity of the current flowing through the nanopore (the amplitude change in the current affected by each type of base is different), and finally, a high-sensitivity electronic device detects these changes to identify the passed bases. At present, protein pores are used as nanopores for sequencing, and porins are mainly derived from *Escherichia coli.*

Currently, the nanoporin is limited, so it is necessary to develop an alternative nanoporin to achieve the nanopore sequencing technology. The porin is also closely related to sequencing precision, and the porin is also involved in a mode change in the interaction with a rate-controlling protein. Therefore, further optimizing the stability of an interaction interface between the porin and the rate-controlling protein has a positive effect on improving the consistency and stability of sequencing data. The accuracy rate of the nanopore sequencing technology also needs to be improved, and therefore, it is necessary to develop an improved nanoporin to further improve the resolution of the nanopore sequencing.

### SUMMARY

In order to solve the problems described above, embodiments of the present invention are intended to provide an alternative mutant of a porin monomer, a protein pore comprising same, and use thereof.

In a first aspect, embodiments of the present invention provide a mutant of a porin monomer, wherein an amino acid of the mutant of the porin monomer comprises or consists of a sequence set forth in SEQ ID NO: 1 or a sequence having at least 99%, 98%, 97%, 96%, 95%, 90%, 80%, 70%, 60%, or 50% identity thereto, and the amino acid of the mutant of the porin monomer comprises mutations at one or more of positions corresponding to A74, P75, G76, N77, A78, T79, N80, and F81 of SEQ ID NO: 1.

Preferably, the amino acid of the mutant of the porin monomer comprises: (1) an insertion, a deletion, and/or a substitution of an amino acid at one or more of positions corresponding to A74, P75, G76, N77, A78, T79, N80, and F81 of SEQ ID NO: 1; or (2) an insertion, a deletion, and/or a substitution of an amino acid at one or more of positions corresponding to A74, P75, G76, N77, A78, T79, N80, F81, S82, and T83 of SEQ ID NO: 1.

In one embodiment, the amino acid mutation of the mutant of the porin monomer is selected from the group consisting of:
(a) mutations from amino acids APGNATNF corresponding to positions 74-81 of SEQ ID NO: 1 to M₁M₂M₃M₄M₅M₆, wherein M₁ is selected from 0 to 1 of P; M₂ is selected from 0 to 3 of Y, F, and W; M₃ is selected from 0 to 1 of P; M₄ is selected from 0 to 5 of A, G, V, L, and I; M₅ is selected from 0 to 4 of N, D, E, and Q; M₆ is selected from 0 to 5 of A, G, V, L, and I;
(b) mutations from amino acids APGNATNFST corresponding to positions 74-83 of SEQ ID NO: 1 to M₇M₈M₉M₁₀M₁₁M₁₂ M₁₃M₁₄, wherein M₇ is selected from 0 to 5 of A, G, V, L, and I; M₈ is selected from 0 to 3 of H, K, and R; M₉ is selected from 0 to 1 of P; M₁₀ is selected from 0 to 5 of A, G, V, L, and I; M₁₁ is selected from 0 to 5 of A, G, V, L, and I; M₁₂ is selected from 0 to 5 of T, S, C, U, and M; M₁₃ is selected from 0 to 5 of S, C, U, T, and M; M₁₄ is selected from 0 to 1 of P;
(c) mutations from the amino acids APGNATNFST corresponding to positions 74-83 of SEQ ID NO: 1 to M₁₅M₁₆M₁₇M₁₈M₁₉M₂₀M₂₁M₂₂, wherein M₁₅ is selected from 0 to 1 of P; M₁₆ is selected from 0 to 5 of A, G, V, L, and I; M₁₇ is selected from 0 to 5 of A, G, V, L, and I; M₁₈ is selected from 0 to 5 of S, C, U, T, and M; M₁₉ is selected from 0 to 5 of S, C, U, T, and M; M₂₀ is selected from 0 to 5 of L, G, A, V, and I; M₂₁ is selected from 0 to 5 of S, C, U, T, and M; M₂₂ is selected from 0 to 1 of P; and
(d) mutations from the amino acids APGNATNFST corresponding to positions 74-83 of SEQ ID NO: 1 to M₂₃M₂₄M₂₅M₂₆M₂₇M₂₈M₂₉M₃₀, wherein M₂₃ is selected from 0 to 1 of P; M₂₄ is selected from 0 to 3 of Y, F, and W; M₂₅ is selected from 0 to 1 of P; M₂₆ is selected from 0 to 5 of A, G, V, L, and I; M₂₇ is selected from 0 to 4 of N, D, E, and Q; M₂₈ is selected from 0 to 5 of A, G, V, L, and I; M₂₉ is selected from 0 to 5 of S, C, U, T, and M; M₃₀ is selected from 0 to 1 of P.

In one embodiment, the amino acid mutation of the mutant of the porin monomer is selected from the group consisting of:
(a) mutations from the amino acids APGNATNF corresponding to positions 74-81 of SEQ ID NO: 1 to M₁M₂M₃M₄M₅M₆, wherein M₁ is selected from P; M₂ is selected from Y, F, or W; M₃ is selected from P; M₄ is selected from A, G, V, L, or I; M₅ is selected from N, D, E, or Q; M₆ is selected from A, G, V, L, or I;
(b) mutations from the amino acids APGNATNFST corresponding to positions 74-83 of SEQ ID NO: 1 to M₇M₈M₉M₁₀M₁₁M₁₂ M₁₃M₁₄, wherein M₇ is selected from A, G, V, L, or I; M₈ is selected from H, K, or R; M₉ is selected from P; M₁₀ is selected from A, G, V, L, or I; M₁₁ is selected from A, G, V, L, or I; M₁₂ is selected from T, S, C, U, or M; M₁₃ is selected from S, C, U, T, or M; M₁₄ is selected from P;
(c) mutations from the amino acids APGNATNFST corresponding to positions 74-83 of SEQ ID NO: 1 to M₁₅M₁₆M₁₇M₁₈M₁₉M₂₀M₂₁M₂₂, wherein M₁₅ is selected from P; M₁₆ is selected from A, G, V, L, or I; M₁₇ is selected from A, G, V, L, or I; M₁₈ is selected from S, C, U, T, or M; M₁₉ is selected from S, C, U, T, or M; M₂₀ is selected from L, G, A, V, or I; M₂₁ is selected from S, C, U, T, or M; M₂₂ is selected from P; and
(d) mutations from the amino acids APGNATNFST corresponding to positions 74-83 of SEQ ID NO: 1 to M₂₃M₂₄M₂₅M₂₆M₂₇M₂₈M₂₉M₃₀, wherein M₂₃ is selected from P; M₂₄ is selected from Y, F, or W; M₂₅ is selected from P; M₂₆ is selected from A, G, V, L, or I; M₂₇ is selected from N, D, E, or Q; M₂₅ is selected from A, G, V, L, or I; M₂₉ is selected from S, C, U, T, or M; M₃₀ is selected from P.

In one embodiment, the amino acid mutation of the mutant of the porin monomer is selected from the group consisting of:
(a) mutations from the amino acids APGNATNF corresponding to positions 74-81 of SEQ ID NO: 1 to PYPANA;
(b) mutations from the amino acids APGNATNFST corresponding to positions 74-83 of SEQ ID NO: 1 to AHPAATSP;
(c) mutations from the amino acids APGNATNFST corresponding to positions 74-83 of SEQ ID NO: 1 to PAASSLSP; and
(d) mutations from the amino acids APGNATNFST corresponding to positions 74-83 of SEQ ID NO: 1 to PYPANASP.

In a second aspect, embodiments of the present invention provide a protein pore comprising at least one mutant of the porin monomer.

In a third aspect, embodiments of the present invention provide a complex for characterizing a target analyte, which comprises the protein pore and a rate-controlling protein bound thereto.

In a fourth aspect, embodiments of the present invention provide a nucleic acid encoding the mutant of the porin monomer, the protein pore, or the complex.

In a fifth aspect, embodiments of the present invention provide a vector or a genetically engineered host cell comprising the nucleic acid.

In a sixth aspect, embodiments of the present invention provide use of the mutant of the porin monomer or the protein pore, the complex, the nucleic acid, or the vector or host cell thereof in the detection of the presence, absence, or one or more characteristics of a target analyte or in the preparation of a product for detecting the presence, absence, or one or more characteristics of a target analyte.

In a seventh aspect, embodiments of the present invention provide a method for producing a protein pore or a polypeptide thereof, comprising transforming the host cell with the vector, and inducing the host cell to express the protein pore or the polypeptide thereof.

In an eighth aspect, embodiments of the present invention provide a method for determining the presence, absence, or one or more characteristics of a target analyte, comprising:
a. contacting the target analyte with the protein pore, the complex, or the protein pore in the complex, such that the target analyte moves relative to the protein pore; and
b. acquiring one or more measurements when the target analyte moves relative to the protein pore, thereby determining the presence, absence, or one or more characteristics of the target analyte.

In one embodiment, the method comprises: the target analyte interacting with the protein pore present in a membrane, such that the target analyte moves relative to the protein pore.

In one embodiment, the target analyte is a nucleic acid molecule.

In one embodiment, the method for determining the presence, absence, or one or more characteristics of a target analyte comprises coupling the target analyte to a membrane; and the target analyte interacting with the protein pore present in the membrane, such that the target analyte moves relative to the protein pore.

In a ninth aspect, embodiments of the present invention provide a kit for determining the presence, absence, or one or more characteristics of a target analyte, comprising the mutant of the porin monomer, the protein pore, the complex, the nucleic acid, or the vector or host cell, and a component of the membrane.

In a tenth aspect, embodiments of the present invention provide a device for determining the presence, absence, or one or more characteristics of a target analyte, comprising the protein pore or the complex, and the membrane.

In one embodiment, the target analyte includes a polysaccharide, a metal ion, an inorganic salt, a polymer, an amino acid, a peptide, a protein, a nucleotide, an oligonucleotide, a polynucleotide, a dye, a drug, a diagnostic agent, an explosive, or an environmental contaminant;
preferably, the target analyte comprises a polynucleotide;
more preferably, the polynucleotide comprises DNA or RNA; and/or, the one or more characteristics are selected from (i) a length of the polynucleotide; (ii) an identity of the polynucleotide; (iii) a sequence of the polynucleotide; (iv) a secondary structure of the polynucleotide; and (v) whether the polynucleotide is modified; and/or, the rate-controlling protein in the complex comprises a polynucleotide binding protein.

### BRIEF DESCRIPTION OF THE DRAWINGS

The drawings described are only schematic rather than restrictive.
FIG. 1 shows the basic working principle of a nanopore according to one embodiment.
FIG. 2 shows a schematic diagram of DNA sequencing according to one embodiment.
FIG. 3 shows a corresponding pore-blocking signal when a nucleotide passes through a protein pore according to one embodiment.
FIGs. 4A and 4B show a channel surface structure and a ribbon diagram model of a wild-type protein pore according to one embodiment. FIG. 4A is a surface structure model, and FIG. 4B is a ribbon structure model.
FIG. 5 shows the amino acid residue distribution in a constriction zone and constriction zone diameters of a wild-type protein pore according to one embodiment.
FIG. 6A shows a surface potential diagram of a wild-type protein monomer according to one embodiment.
FIG. 6B shows a ribbon model of the monomer and a stick model of the amino acid residue distribution in a constriction zone of the monomer according to one embodiment.
FIG. 7A shows amino acid residue distribution characteristics in a constriction zone and constriction zone diameters of mutant pore 1 according to one embodiment.
FIG. 7B shows a ribbon diagram of FIG. 7A.
FIG. 8 shows a cartoon representation of the mutant pore 1 based on homologous modeling according to one embodiment.
FIG. 9 shows the structure of a DNA construct, BS7-4C3-SE1, according to one embodiment.
FIG. 10 shows the structure of a DNA construct, BS7-4C3-PLT, according to one embodiment.
FIG. 11A shows an opening current and gated features of the mutant pore 1 at a voltage of ±180 mV according to one embodiment.
FIG. 11B shows a scenario in which a nucleic acid passes through the pore of the mutant pore 1 at a voltage of +180 mV according to one embodiment.
FIGs. 12A, 12B, 12C, and 12D show example current trajectories when helicase Mph-MP1-E105C/A362C controls the translocation of the DNA construct BS7-4C3-SE1 through the mutant pore 1 according to one embodiment.
FIG. 13 is an enlarged area display of a single signal of the embodiment in FIG. 12B.
FIG. 14A shows an opening current and gated features of protein pore mutant 2 at a voltage of ±180 mV according to one embodiment.
FIG. 14B shows a scenario in which a nucleic acid passes through the pore of the protein pore mutant 2 at a voltage of +180 mV according to one embodiment.
FIGs. 15A and 15B show example current trajectories when the helicase Mph-MP1-E105C/A362C controls the translocation of the DNA construct BS7-4C3-PLT through the mutant pore 2 according to one embodiment.
FIG. 16 is an enlarged area display of a single signal of the embodiment in FIG. 15A.
FIG. 17A shows an opening current and gated features of protein pore mutant 3 at a voltage of ±180 mV according to one embodiment.
FIG. 17B shows a scenario in which a nucleic acid passes through the pore of the protein pore mutant 3 at a voltage of +180 mV according to one embodiment.
FIGs. 18A, 18B, 18C, 18D, 18E, and 18F show example current trajectories when the helicase Mph-MP1-E105C/A362C controls the translocation of the DNA construct BS7-4C3-PLT through the mutant pore 3 according to one embodiment.
FIG. 19 is an enlarged area display of a single signal of the embodiment in FIG. 18B.
FIG. 20A shows an opening current and gated features of protein pore mutant 4 at a voltage of ±180 mV according to one embodiment.
FIG. 20B shows a scenario in which a nucleic acid passes through the pore of the protein pore mutant 4 at a voltage of +180 mV according to one embodiment.
FIGs. 21A, 21B, 21C, 21D, 21E, and 21F show example current trajectories when the helicase Mph-MP1-E105C/A362C controls the translocation of the DNA construct BS7-4C3-PLT through the mutant pore 4 according to one embodiment.
FIG. 22 is an enlarged area display of a single signal of the embodiment in FIG. 21.
FIG. 23 shows SDS-PAGE electrophoresis results of the mutant 1 according to one embodiment.

### DETAILED DESCRIPTION

It should be understood that unused applications of the disclosed products and methods may be adapted according to the particular needs in the art. It should also be understood that the terms used herein are for the purpose of describing particular embodiments of the present invention only, and are not intended to be limiting.

In addition, as used in this specification and the claims, the singular forms "a", "an", and "the" include plural referents, unless otherwise specified clearly in the context. For example, reference to "a nucleotide" includes two or more nucleotides, and reference to "a helicase" includes two or more helicases.

As used herein, the term "comprising" means that any of the listed elements must be included, and that other elements may also optionally be included. "Consisting of..." means excluding all unlisted elements. Embodiments defined by each of these terms are within the scope of the present invention.

As used herein, a "nucleotide sequence", "DNA sequence", or "nucleic acid molecule" refers to a polymeric form of nucleotides (ribonucleotides or deoxyribonucleotides) of any length. The term only refers to the primary structure of the molecule. Thus, the term includes double-stranded and single-stranded DNA and RNA.

The term "nucleic acid" as used herein refers to a single-stranded or double-stranded covalently linked nucleotide sequence in which the 3' and 5' ends on each nucleotide are linked by phosphodiester bonds. A nucleotide may consist of deoxyribonucleotide bases or ribonucleotide bases. Nucleic acids may include DNA and RNA, and may be prepared synthetically *in vitro* or isolated from natural sources. Nucleic acids may further include modified DNA or RNA, such as methylated DNA or RNA, or RNA that has been subjected to post-translational modification, for example, 5'-capping with 7-methylguanosine, and 3'-end processing, such as cleavage and polyadenylation, and splicing. Nucleic acids may also include synthetic nucleic acids (XNA), such as a hexitol nucleic acid (HNA), a cyclohexene nucleic acid (CeNA), a threose nucleic acid (TNA), a glycerol nucleic acid (GNA), a locked nucleic acid (LNA), and a peptide nucleic acid (PNA). The size of a nucleic acid (or polynucleotide) is generally expressed in terms of the number of base pairs (bp) of a double-stranded polynucleotide, or in the case of a single-stranded polynucleotide, in terms of the number of nucleotides (nt). One thousand bp or nt equals one kilobase pair (kb). Polynucleotides of less than about 40 nucleotides in length are generally referred to as "oligonucleotides" and may comprise primers for use in DNA manipulation, for example, by polymerase chain reaction (PCR).

A polynucleotide, such as a nucleic acid, is a macromolecule comprising two or more nucleotides. The polynucleotide or nucleic acid may comprise any combination of any nucleotides. The nucleotides may be naturally occurring or synthetic. One or more nucleotides in the polynucleotide may be oxidized or methylated. One or more nucleotides in the polynucleotide may be damaged. For example, the polynucleotide may comprise a pyrimidine dimer. This dimer is generally associated with the damage caused by ultraviolet light and is the major cause of cutaneous melanoma. One or more nucleotides in the polynucleotide may be modified, for example, with a conventional label or tag. The polynucleotide may comprise one or more nucleotides that are abasic (i.e., lack nucleobases), or lack nucleobases and sugars (i.e., C3).

The nucleotides in the polynucleotide may be linked to each other in any manner. The nucleotides are generally linked by glycosyl and phosphate groups thereof, as in the nucleic acid. The nucleotides may be linked by nucleobases thereof, as in the pyrimidine dimer.

The polynucleotide may be single-stranded or double-stranded. At least a portion of the polynucleotide is preferably double-stranded. The polynucleotide may be a nucleic acid, such as deoxyribonucleic acid (DNA) or ribonucleic acid (RNA). The polynucleotide may comprise an RNA strand that is hybridized to a DNA strand. The polynucleotide may be any synthetic nucleic acid known in the art, such as a peptide nucleic acid (PNA), a glycerol nucleic acid (GNA), a threose nucleic acid (TNA), a locked nucleic acid (LNA), or other synthetic polymers having nucleotide side chains. The PNA backbone is composed of repeating N-(2-aminoethyl)-glycine units linked by peptide bonds. The GNA backbone is composed of repeating ethylene glycol units linked by phosphodiester bonds. The TNA backbone is composed of repeating threose sugars linked together by phosphodiester bonds. LNA is formed from the ribonucleic acid described above and has an additional bridging structure linking the 2' oxygen and the 4' carbon in the ribose moiety. Bridged nucleic acids (BNAs) are modified RNA nucleotides. They may also be referred to as restricted or inaccessible RNA13BNA monomers that may contain a 5-, 6-, or even 7-membered bridging structure and have a "fixed" C3'-endo sugar puckering structure. The bridging structure is synthetically introduced into the position 2',4' of the ribose to produce the 2',4'-BNA monomer.

The polynucleotide is most preferably ribonucleic acid (RNA) or deoxyribonucleic acid (DNA). The polynucleotide may be of any length. For example, the polynucleotide may be of at least 10, at least 50, at least 100, at least 150, at least 200, at least 250, at least 300, at least 400, or at least 500 nucleotides or nucleotide pairs in length. The polynucleotide may be of 1000 or more nucleotides or nucleotide pairs, 5000 or more nucleotides or nucleotide pairs, or 100000 or more nucleotides or nucleotide pairs in length.

Any number of polynucleotides may be studied. For example, the methods of the embodiments may involve the characterization of 2, 3, 4, 5, 6, 7, 8, 9, 10, 20, 30, 50, 100, or more polynucleotides. If two or more polynucleotides are characterized, they may be different polynucleotides or the same polynucleotide.

The polynucleotides may be naturally occurring or synthetic. For example, the method may be used to verify the sequence of the prepared oligonucleotides. The method is generally performed *in vitro.*

In the context of the present disclosure, the term "amino acid" is used in its broadest sense and is meant to include organic compounds containing amine (NH₂) and carboxyl (COOH) functional groups as well as side chains unique to each amino acid (e.g., R groups). In some embodiments, the amino acid refers to naturally occurring Lα-amino acids or residues. Commonly used single and three-letter abbreviations for the naturally occurring amino acids as used herein are as follows: A = Ala; C = Cys; D = Asp; E = Glu; F = Phe; G = Gly; H = His; I = Ile; K = Lys; L = Leu; M = Met; N = Asn; P = Pro; Q = Gln; R = Arg; S = Ser; T = Thr; V = Val; W = Trp; and Y = Tyr (Lehninger, A. L., (1975) BioChemistry, 2nd edition, pages 71-92, Worth Publishers, New York). The common term "amino acid" further includes D-amino acids, retro-inverso amino acids, chemically modified amino acids (such as amino acid analogs), naturally occurring amino acids that are not generally incorporated into proteins (such as norleucine), and chemically synthesized compounds (such as β-amino acids) that have properties to be characteristic of amino acids known in the art. For example, included within the definition of amino acids are analogs or mimetics of phenylalanine or proline that allow the same conformational restriction on peptide compounds as native Phe or Pro. Such analogs and mimetics are referred to herein as "functional equivalents" of the corresponding amino acids. Other examples of amino acids are listed in Roberts and Vellaccio, The Peptides: Analysis, Synthesis, Biology, edited by Gross and Meiehofer, Volume 5, page 341, Academic Press, Inc., N. Y. 1983, which is incorporated herein by reference.

The terms "protein," "polypeptide", and "peptide" are further used interchangeably herein and refer to a polymer of amino acid residues as well as a variant and synthetic analog of amino acid residues. Thus, these terms apply to amino acid polymers in which one or more amino acid residues are synthetic and non-naturally occurring amino acids, such as chemical analogs of corresponding naturally occurring amino acids, as well as to naturally occurring amino acid polymers. The polypeptide may also be subjected to maturation or post-translational modification processes, which may include, but are not limited to: glycosylation, proteolytic cleavage, lipidation, signal peptide cleavage, propeptide cleavage, phosphorylation, and the like.

"Homologs" of a protein encompass peptides, oligopeptides, polypeptides, proteins, and enzymes having amino acid substitutions, deletions, and/or insertions relative to the unmodified or wild-type protein in discussion and having similar biological and functional activities to the unmodified protein from which they are derived. As used herein, the term "amino acid identity" refers to the degree to which sequences are identical on an amino acid-to-amino acid basis in a comparison window. Thus, the "percent sequence identity" is calculated by the following steps: comparing two optimally aligned sequences in a comparison window; determining the number of positions in which amino acid residues (e.g., Ala, Pro, Ser, Thr, Gly, Val, Leu, Ile, Phe, Tyr, Trp, Lys, Arg, His, Asp, Glu, Asn, Gln, Cys, and Met) are identical in the two sequences to yield the number of matched positions; dividing the number of matched positions by the total number of positions in the comparison window (i.e., the window size); and multiplying the result by 100 to yield the percent sequence identity.

The sequence identity may also be a fragment or portion of a full-length polynucleotide or polypeptide. Thus, a sequence may have only 50% overall sequence identity to a full-length reference sequence, but the sequence of a particular region, domain, or subunit may have 80%, 90%, or up to 99% sequence identity to the reference sequence.

The term "wild-type" refers to a gene or gene product isolated from a naturally occurring source. The wild-type gene is a gene most commonly observed in a population and is therefore arbitrarily designed as the "normal" or "wild-type" form of the gene. Conversely, the term "modified," "mutation", or "variant" refers to a gene or gene product that exhibits sequence modification (e.g., substitution, truncation, or insertion), post-translational modification, and/or functional properties (e.g., altered characteristics) compared to the wild-type gene or gene product. It is noted that naturally occurring mutants may be isolated. These mutants are identified by the fact that they have altered characteristics compared to the wild-type gene or gene product. Methods for introducing or substituting naturally occurring amino acids are well known in the art. For example, methionine (M) may be substituted with arginine (R) by replacing the codon of methionine (ATG) with the codon of arginine (CGT) at the relevant position in the polynucleotide encoding the mutated monomer. Methods for introducing or substituting non-naturally occurring amino acids are also well known in the art. For example, the non-naturally occurring amino acids may be introduced by including synthetic aminoacyl-tRNA in the IVTT system for expressing the mutated monomer. Alternatively, the non-naturally occurring amino acids may be introduced by expressing the mutated monomer in Thermodesulfovibrio sp. N1, which is auxotrophic for particular amino acids in the presence of synthetic (i.e., non-naturally occurring) analogs of those particular amino acids. If the mutated monomers are generated using partial peptide synthesis, they may also be generated by naked linkage. A conservative substitution is the replacement of amino acids by other amino acids having similar chemical structures, similar chemical properties, or similar side chain volumes. The amino acids introduced may have similar polarity, hydrophilicity, hydrophobicity, basicity, acidity, neutrality, or charge to the amino acids they replace. Alternatively, the conservative substitution may be the introduction of another aromatic or aliphatic amino acid in place of a pre-existing aromatic or aliphatic amino acid. Conservative amino acid changes are well known in the art and may be selected in accordance with the properties of the 20 major amino acids defined in Table 1 below. In the case of amino acids with similar polarity, this may also be determined with reference to the hydrophilicity scale of the amino acid side chains in Table 2.

**Table 1. Chemical properties of amino acids**

| | | | |
|---|---|---|---|
| Ala, A | Aliphatic, hydrophobic, and neutral | Met, M | Hydrophobic and neutral |
| Cys, C | Polar, hydrophobic, and neutral | Asn, N | Polar, hydrophilic, and neutral |
| Asp, D | Polar, hydrophilic, and charged (-) | Pro, P | Hydrophobic and neutral |
| Glu, E | Polar, hydrophilic, and charged (-) | Gln, Q | Polar, hydrophilic, and neutral |
| Phe, F | Aromatic, hydrophobic, and neutral | Arg, R | Polar, hydrophilic, and charged (+) |
| Gly, G | Aliphatic and neutral | Ser, S | Polar, hydrophilic, and neutral |
| His, H | Aromatic, polar, hydrophilic, and charged (+) | Thr, T | Polar, hydrophilic, and neutral |
| Ile, I | Aliphatic, hydrophobic, and neutral | Val, V | Aliphatic, hydrophobic, and neutral |
| Lys, K | Polar, hydrophilic, and charged (+) | Trp, W | Aromatic, hydrophobic, and neutral |
| Leu, L | Aliphatic, hydrophobic, and neutral | Tyr, Y | Aromatic, polar, and hydrophobic |

**Table 2. Hydrophilicity scale**

| Side chain | Hydrophilicity |
|---|---|
| Ile, I | 4.5 |
| Val, V | 4.2 |
| Leu, L | 3.8 |
| Phe, F | 2.8 |
| Cys, C | 2.5 |
| Met, M | 1.9 |
| Ala, A | 1.8 |
| Gly, G | -0.4 |
| Thr, T | -0.7 |
| Ser, S | -0.8 |
| Trp, W | -0.9 |
| Tyr, Y | -1.3 |
| Pro, P | -1.6 |
| His, H | -3.2 |
| Glu, E | -3.5 |
| Gln, Q | -3.5 |
| Asp, D | -3.5 |
| Asn, N | -3.5 |
| Lys, K | -3.9 |
| Arg, R | -4.5 |

It is well known that conservative substitutions of amino acids with similar properties between each other, such as those in Table 3, do not generally affect the activity of peptide sequences.

**Table 3. Conservative amino acid substitutions**

| Type | Amino acid |
|---|---|
| Aliphatic | Glycine (G), alanine (A), valine (V), leucine (L), and isoleucine (I) |
| Hydrated or sulfur/ selenium-containing | Serine (S), cysteine (C), selenocysteine (U), threonine (T), and methionine (M) |
| Cyclic | Proline (P) |
| Aromatic | Phenylalanine (F), tyrosine (Y), and tryptophan (W) |
| Basic | Histidine (H), lysine (K), and arginine (R) |
| Acidic and amide | Aspartic acid (D), glutamic acid (E), asparagine (N), and glutamine (Q) |

The mutated or modified protein, monomer, or peptide may also be chemically modified in any manner at any site. The mutated or modified monomer or peptide is preferably chemically modified by attachment of the molecule to one or more cysteines (cysteine linkage), attachment of the molecule to one or more lysines, attachment of the molecule to one or more non-natural amino acids, and enzymatic modification of epitopes or terminal modification. Suitable methods for performing such modifications are well known in the art. A mutant of the modified protein, monomer, or peptide may be chemically modified by attachment of any molecule. For example, the mutant of the modified protein, monomer, or peptide may be chemically modified by attachment of a dye or fluorophore. In some embodiments, the mutated or modified monomer or peptide is chemically modified with a molecular adapter that facilitates interaction between a pore comprising a monomer or peptide and a target nucleotide or target polynucleotide sequence. The molecular adapter is preferably a cyclic molecule, a cyclodextrin, a substance capable of hybridizing, a DNA binding agent or intercalator, a peptide or peptide analog, a synthetic polymer, an aromatic planar molecule, a positively charged small molecule, or a small molecule capable of hydrogen bonding.

The presence of the adapter improves the host-guest chemistry of the pore and the nucleotide or polynucleotide sequence, thereby improving the sequencing capability of the pore formed by the mutated monomer. The principles of host-guest chemistry are well known in the art. The adapter has an effect on the physical or chemical properties of the pore, which improves the interaction between the pore and the nucleotide or polynucleotide sequence. The adapter may alter the charge of a barrel or channel of the pore, or specifically interact with or bind to the nucleotide or polynucleotide sequence, thereby facilitating the interaction between the nucleotide or polynucleotide sequence and the pore.

A "protein pore" is a transmembrane protein structure that defines a channel or pore that allows molecules and ions to translocate from one side of the membrane to the other side. The translocation of ionic substances through the pore may be driven by a potential difference applied to either side of the pore. A "nanopore" is a protein pore in which the smallest diameter of the channel through which molecules or ions pass is on the order of nanometers (10⁻⁹ meters). In some embodiments, the protein pore may be a transmembrane protein pore. The transmembrane protein structure of the protein pore may be essentially monomeric or oligomeric. Generally, the pore comprises a plurality of polypeptide subunits arranged around a central axis, thereby forming a protein-lined channel extending substantially perpendicular to the membrane in which the nanopore resides. The number of polypeptide subunits is not limited. Generally, the number of subunits is from 5 to 30, suitably from 6 to 10. Alternatively, the number of subunits is not defined as in the case of perfringolysin or related large membrane pores. The protein subunit portions within the nanopore that form the protein-lined channel generally comprise a secondary structural motif that may include one or more transmembrane β-barrel and/or α-helix portions.

In one embodiment, the protein pore comprises one or more porin monomers. Each porin monomer may be derived from Thermodesulfovibrio sp. N1. In one embodiment, the protein pore comprises one or more mutants of the porin monomer (i.e., one or more mutated monomers of porins).

In one embodiment, the porin is derived from a wild-type protein, wild-type homolog, or mutant thereof in the biological world. The mutant may be a modified porin or a porin mutant. Modifications in the mutants include, but are not limited to, any one or more of the modifications disclosed herein or a combination of the modifications. In one embodiment, the wild-type protein in the biological world is a protein derived from Thermodesulfovibrio sp. N1.

In one embodiment, the porin homolog refers to a polypeptide having at least 99%, 98%, 97%, 96%, 95%, 94%, 93%, 92%, 91%, 90%, 85%, 80%, 75%, 70%, 65%, 60%, 55%, or 50% complete sequence identity to a protein set forth in SEQ ID NO: 1.

In one embodiment, the porin homolog refers to a polynucleotide having at least 99%, 98%, 97%, 96%, 95%, 94%, 93%, 92%, 91%, 90%, 85%, 80%, 75%, 70%, 65%, 60%, 55%, or 50% complete sequence identity to a polynucleotide encoding a protein set forth in SEQ ID NO: 2. The polynucleotide sequence may comprise a sequence that differs from SEQ ID NO: 2 based on the degeneracy of the genetic code.

Polynucleotide sequences may be derived and replicated using standard methods in the art. Chromosomal DNA encoding the wild-type porin may be extracted from pore-producing organisms such as Thermodesulfovibrio sp. N1. A gene encoding the pore subunit may be amplified using PCR comprising specific primers. The amplified sequence may then be subjected to site-directed mutagenesis. Suitable methods for the site-directed mutagenesis are known in the art and include, for example, combine chain reaction. The constructed polynucleotides encoding the embodiments may be prepared using techniques well known in the art, such as those described in Sambrook, J. and Russell, D., (2001) Molecular Cloning A Laboratory Manual, 3rd Edition., Cold Spring Harbor Laboratory Press, Cold Spring Harbor, NY.

The resulting polynucleotide sequences may then be integrated into recombinant replicable vectors, such as cloning vectors. The vectors may be used to replicate the polynucleotides in compatible host cells. Thus, the polynucleotide sequences may be prepared by introducing the polynucleotides into replicable vectors, introducing the vectors into compatible host cells, and allowing the growth of the host cells under conditions that cause the replication of vectors. The vectors may be recovered from the host cells.

### Basic working principle of nanopore or protein pore

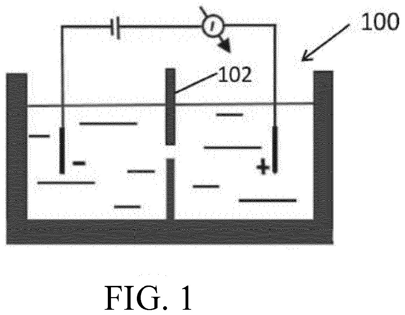

In one embodiment, in a cavity 100 filled with electrolyte, an insulating film 102 having a nanoscale pore divides the cavity into 2 chambers, as shown in FIG. 1. When a voltage is applied to the electrolyte chamber, ions or other small molecule substances pass through the pore under the force of an electric field, resulting in a stable detectable ionic current. By controlling the size and surface characteristics of the nanopore, the applied voltage, and the solution conditions, different types of biomolecules may be detected.

Because the four types of bases, adenine (A), guanine (G), cytosine (C), and thymine (T), which form DNA have different molecular structures and volume sizes, when single-stranded DNA (ssDNA) passes through the nanoscale pore under the drive of a rate-controlling enzyme and the electric field, the difference in the chemical properties of different bases leads to different amplitude changes in the current when it passes through the nanopore or protein pore, thereby obtaining the sequence information about the detected nucleic acid, such as DNA.

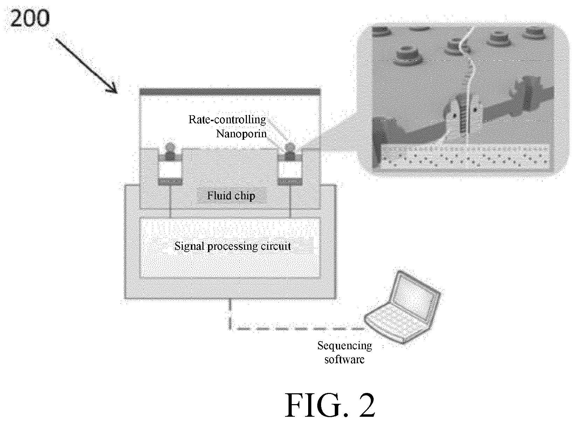

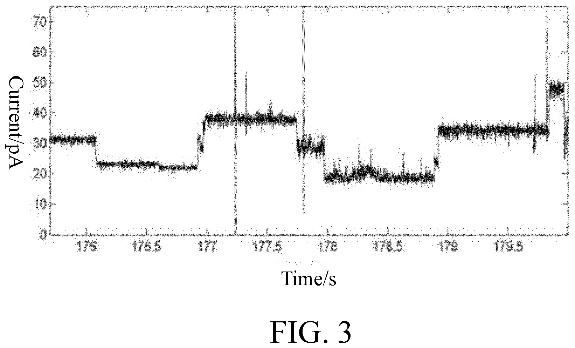

FIG. 2 shows a schematic diagram 200 of DNA sequencing. As shown in FIG. 2, in a typical nanopore/protein pore sequencing experiment, the nanopore is the only channel through which ions on both sides of the phospholipid membrane pass. Rate-controlling proteins, such as polynucleotide binding proteins, act as motor proteins for the nucleic acid molecules, such as DNA, and pull DNA strands to sequentially pass through the nanopore/protein pore in steps of a single nucleotide. Whenever a nucleotide passes through the nanopore/protein pore, a corresponding pore-blocking signal is recorded (FIG. 3). By analyzing the current signals associated with these sequences using a corresponding algorithm, sequence information about the nucleic acid molecules, such as DNA, may be deduced.

In the embodiments, the porin is screened out from different species in nature (mainly bacteria and archaea) by bioinformatics means and from evolutionary perspectives. In one embodiment, the porin is derived from any organism, preferably derived from Thermodesulfovibrio sp. N1, and has a sequence identity of less than 40% to a protein homologous to an *E. coli* amyloid secretion channel (*E. coli* CsgG), preferably a sequence identity of 20%-40%. By sequence analysis, the porin has an intact functional domain. A porin 3D structure model is predicted and analyzed by using a structural biology means, and a channel protein with a proper reading head architecture is selected. Then, candidate channel proteins (or porins) are modified, tested, and optimized by means of genetic engineering, protein engineering, protein directed evolution, computer-aided protein design, and the like, and after several iterations, a plurality of homologous protein mutants, preferably four homologous protein mutants (different homologous protein scaffolds) are obtained, which have different signal characteristics and signal distribution patterns.

The porin in the embodiments may be applied to the fourth generation sequencing technology. In one embodiment, the porin is a nanoporin. In one embodiment, the porin may be applied to solid-state pores for sequencing.

In one embodiment, a new protein scaffold is employed to form a new constriction zone (reading head region) structure, thereby providing a novel mode of action during sequencing. The porins of the embodiments have good jump distribution and recombination efficiency with phospholipid membranes.

In one embodiment, a wild-type porin monomer is modified by gene mutation to form a mutant of the porin monomer. In one embodiment, an amino acid of the mutant of the porin monomer comprises a sequence set forth in SEQ ID NO: 1 or a sequence having at least 99%, 98%, 97%, 96%, 95%, 94%, 93%, 92%, 91%, 90%, 85%, 80%, 75%, 70%, 65%, 60%, 55%, or 50% identity thereto, and the amino acid of the mutant of the porin monomer has mutations corresponding to positions 74-81 or 74-83 of SEQ ID NO: 1.

In one embodiment, the mutation comprises an insertion, a deletion, and/or a substitution of an amino acid. In one embodiment, the mutations at positions 74-81 or 74-83 of SEQ ID NO: 1 are (1) insertions, deletions, and/or substitutions of amino acids at one or more of positions 74-81 of SEQ ID NO: 1; or (2) insertions, deletions, and/or substitutions of amino acids at one or more of positions 74-83 of SEQ ID NO: 1.

In one embodiment, the amino acid of the mutant of the porin monomer has (1) an insertion, a deletion, and/or a substitution of an amino acid at one or more of positions corresponding to A74, P75, G76, N77, A78, T79, N80, and F81 of SEQ ID NO: 1; or (2) an insertion, a deletion, and/or a substitution of an amino acid at one or more of positions corresponding to A74, P75, G76, N77, A78, T79, N80, F81, S82, and T83 of SEQ ID NO: 1.

In one embodiment, the amino acid of the mutant of the porin monomer has mutations only at positions 74-81 (i.e., A74, P75, G76, N77, A78, T79, N80, and F81) corresponding to SEQ ID NO: 1.

In one embodiment, the amino acid of the mutant of the porin monomer has insertions, deletions, and/or substitutions of amino acids only at one or more of positions 74-81 (i.e., A74, P75, G76, N77, A78, T79, N80, and F81) corresponding to SEQ ID NO: 1.

In one embodiment, the position corresponding to SEQ ID NO: 1 means that regardless of whether the sequence numbering is changed by insertions or deletions of amino acids or by adopting a sequence having identity, the relative position is unchanged and the sequence numbering of SEQ ID NO: 1 may be still used. For example, A74 corresponding to SEQ ID NO: 1 may be mutated to A74P, and even if the sequence numbering of SEQ ID NO: 1 is changed or a sequence having the identity as defined herein to SEQ ID NO: 1 is adopted, the amino acid A at position 74 corresponding to SEQ ID NO: 1 (even if this amino acid is not at position 74 in another sequence) may also be mutated to P, and still be within the scope of the present invention.

In one embodiment, the amino acid of the mutant of the porin monomer consists of a sequence set forth in SEQ ID NO: 1 or a sequence having at least 99%, 98%, 97%, 96%, 95%, 94%, 93%, 92%, 91%, 90%, 85%, 80%, 75%, 70%, 65%, 60%, 55%, or 50% identity thereto, and the amino acid of the porin has mutations corresponding to positions 74-81 or 74-83 of SEQ ID NO: 1.

In one embodiment, the sequence set forth in SEQ ID NO: 1 of the porin monomer is derived from Thermodesulfovibrio sp. N1. The nucleotide sequence encoding the amino acid of SEQ ID NO: 1 is set forth in SEQ ID NO: 2.

In one embodiment, the mutant of the protein monomer has mutations at positions 74-81 or at positions 74-83 in the sequence set forth in SEQ ID NO: 1.

In one embodiment, the amino acids corresponding to positions 74-81 of SEQ ID NO: 1 are mutated to or substituted with M₁M₂M₃M₄M₅M₆, wherein M₁ is selected from P; M₂ is selected from Y, F, or W; M₃ is selected from P; M₄ is selected from A, G, V, L, or I; M₅ is selected from N, D, E, or Q; M₆ is selected from A, G, V, L, or I.

In one embodiment, the amino acids corresponding to positions 74-83 of SEQ ID NO: 1 are mutated to or substituted with M₇M₈M₉M₁₀M₁₁M₁₂M₁₃M₁₄, wherein M₇ is selected from A, G, V, L, or I; M₈ is selected from H, K, or R; M₉ is selected from P; M₁₀ is selected from A, G, V, L, or I; M₁₁ is selected from A, G, V, L, or I; M₁₂ is selected from T, S, C, U, or M; M₁₃ is selected from S, C, U, T, or M; M₁₄ is selected from P.

In one embodiment, the amino acids corresponding to positions 74-83 of SEQ ID NO: 1 are mutated to or substituted with M₁₅M₁₆M₁₇M₁₈M₁₉M₂₀ M₂₁M₂₂, wherein M₁₅ is selected from P; M₁₆ is selected from A, G, V, L, or I; M₁₇ is selected from A, G, V, L, or I; M₁₈ is selected from S, C, U, T, or M; M₁₉ is selected from S, C, U, T, or M; M₂₀ is selected from L, G, A, V, or I; M₂₁ is selected from S, C, U, T, or M; M₂₂ is selected from P.

In one embodiment, the amino acids corresponding to positions 74-83 of SEQ ID NO: 1 are mutated to or substituted with M₂₃M₂₄M₂₅M₂₆M₂₇M₂₈ M₂₉M₃₀, wherein M₂₃ is selected from P; M₂₄ is selected from Y, F, or W; M₂₅ is selected from P; M₂₆ is selected from A, G, V, L, or I; M₂₇ is selected from N, D, E, or Q; M₂₅ is selected from A, G, V, L, or I; M₂₉ is selected from S, C, U, T, or M; M₃₀ is selected from P.

In one embodiment, in the mutant of the porin monomer, the amino acid mutation is selected from the group consisting of:
(a) mutations or substitutions from the amino acids APGNATNF corresponding to positions 74-81 of SEQ ID NO: 1 to PYPANA;
(b) mutations or substitutions from the amino acids APGNATNFST corresponding to positions 74-83 of SEQ ID NO: 1 to AHPAATSP;
(c) mutations or substitutions from the amino acids APGNATNFST corresponding to positions 74-83 of SEQ ID NO: 1 to PAASSLSP; and
(d) mutations or substitutions from the amino acids APGNATNFST corresponding to positions 74-83 of SEQ ID NO: 1 to PYPANASP.

In one embodiment, the mutant of the porin monomer comprises or consists of an amino acid sequence set forth in SEQ ID NO: 24, SEQ ID NO: 26, SEQ ID NO: 27, or SEQ ID NO: 28.

In one embodiment, the protein pore comprises at least one mutant of the porin monomer (or porin-mutated monomer). In one embodiment, the protein pore comprises at least two, three, four, five, six, seven, eight, nine, ten, or more mutants of the porin monomer. In one embodiment, the protein pore comprises at least two mutants of the porin monomer, and the mutants of the porin monomer may be identical or different. In one embodiment, the protein pore comprises two or more mutants of the porin monomer; preferably, the two or more mutants of the monomer are identical. In one embodiment, the protein pore has a pore channel diameter of a constriction zone of 0.7 nm to 2.2 nm, 0.9 nm to 1.6 nm, 1.4 nm to 1.6 nm, or 12.7 Å to 22.2 Å.

Provided is use of the mutant of the porin monomer or the protein pore comprising same in the detection of the presence, absence, or one or more characteristics of a target analyte. In one embodiment, the mutant of the porin monomer or the protein pore is used to detect the sequence of nucleic acid molecules, or to characterize the sequence of polynucleotides, such as sequencing polynucleotides, because they may distinguish different nucleotides with high sensitivity. The mutant of the porin monomer or the protein pore comprising same may distinguish four types of nucleotides in DNA and RNA, and even may distinguish between methylated and unmethylated nucleotides, with unexpectedly high resolution. The mutant of the porin monomer or the protein pore shows almost complete separation from all four types of DNA/RNA nucleotides. Deoxycytidine monophosphate (dCMP) and methyl-dCMP are further distinguished based on the dwell time in the protein pore and the current flowing through the protein pore.

The mutant of the porin monomer or the protein pore may also distinguish between different nucleotides under a range of conditions. In particular, the mutant of the porin monomer or the protein pore distinguishes nucleotides under conditions that are favorable for nucleic acid characterization such as sequencing. By altering the applied potential, salt concentrations, buffers, temperature, and the presence of additives such as urea, betaine and DTT, the extent to which the mutant of the porin monomer or the protein pore distinguishes between different nucleotides may be controlled. This allows the functions of the mutant of the porin monomer or the protein pore to be finely regulated and controlled, especially during sequencing. The mutant of the porin monomer or the protein pore may also be used to identify polynucleotide polymers through the interaction with one or more monomers rather than on a nucleotide-by-nucleotide basis.

The mutant of the porin monomer or the protein pore may be isolated, substantially isolated, purified, or substantially purified. The mutant of the porin monomer or the protein pore of the embodiments is isolated or purified if it is completely free of any other components, such as liposomes or other protein pores/porins. The mutant of the porin monomer or the protein pore is substantially isolated if it is mixed with a carrier or diluent that does not interfere with its intended use. For example, the mutant of the porin monomer or the protein pore is substantially isolated or substantially purified if it is present in a form comprising less than 10%, less than 5%, less than 2%, or less than 1% of other components, such as triblock copolymers, liposomes, or other protein pores/porins. Alternatively, the mutant of the porin monomer or the protein pore may be present in a membrane.

For example, the membrane is preferably an amphiphilic layer. The amphiphilic layer is a layer formed of amphiphilic molecules, for example, phospholipids, which have hydrophilicity and lipophilicity. The amphiphilic molecules may be synthetic or naturally occurring. The amphiphilic layer may be a monolayer or a bilayer. The amphiphilic layer is generally planar. The amphiphilic layer may be curved. The amphiphilic layer may be supported. The membrane may be a lipid bilayer. The lipid bilayer is formed of two opposing layers of lipids. The two layers of the lipids are arranged in such way that their hydrophobic tail groups face each other to form a hydrophobic interior. The hydrophilic head groups of the lipids face outward towards the aqueous environment on each side of the bilayer. The membrane comprises a solid layer. The solid layer may be formed from organic and inorganic materials. If the membrane comprises a solid layer, the pore is generally present in the amphiphilic membrane or in a layer comprised within the solid layer, for example, in holes, wells, gaps, channels, grooves, or slits within the solid layer.

### Characterization of analytes

Embodiments provide a method for determining the presence, absence, or one or more characteristics of a target analyte. The method involves contacting the target analyte with a mutant of a porin monomer or a protein pore, such that the target analyte moves relative to, e.g., through, the mutant of the porin monomer or the protein pore, and acquiring one or more measurements when the target analyte moves relative to the mutant of the porin monomer or the protein pore, thereby determining the presence, absence, or one or more characteristics of the target analyte. The target analyte may also be referred to as a template analyte or analyte of interest.

The target analyte is preferably a polysaccharide, a metal ion, an inorganic salt, a polymer, an amino acid, a peptide, a polypeptide, a protein, a nucleotide, an oligonucleotide, a polynucleotide, a dye, a drug, a diagnostic agent, an explosive, or an environmental contaminant. The method may involve determining the presence, absence, or one or more characteristics of two or more target analytes of the same class, e.g., two or more proteins, two or more nucleotides, or two or more drugs. Alternatively, the method may involve determining the presence, absence, or one or more characteristics of two or more target analytes of different classes, e.g., one or more proteins, one or more nucleotides, and one or more drugs.

The method comprises contacting the target analyte with a mutant of a porin monomer or a protein pore, such that the target analyte moves through the mutant of the porin monomer or the protein pore. The protein pore generally comprises at least 1, at least 2, at least 3, at least 4, at least 5, at least 6, at least 7, at least 8, at least 9, or at least 10 porin-mutated monomers, for example, 7, 8, 9, or 10 monomers. The protein pore comprises identical monomers or different porin monomers, preferably 8 or 9 identical monomers. One or more, such as 2, 3, 4, 5, 6, 7, 8, 9, or 10, of the monomers are preferably chemically modified as discussed above. In one embodiment, the amino acid of each monomer comprises SEQ ID NO: 1 and mutants thereof as described above. In one embodiment, the amino acid of each monomer consists of SEQ ID NO: 1 and mutants thereof as described above.

The method of the embodiments may measure two, three, four, five, or more characteristics of a polynucleotide. The one or more characteristics are preferably selected from (i) a length of the polynucleotide, (ii) an identity of the polynucleotide, (iii) a sequence of the polynucleotide, (iv) a secondary structure of the polynucleotide, and (v) whether the polynucleotide is modified. In one embodiment, any combination of (i) to (v) may be measured.

For (i), the length of the polynucleotide may be measured, for example, by determining the number of interactions between the polynucleotide and the mutant of the protein monomer/protein pore or the duration time of the interaction between the polynucleotide and the mutant of the protein monomer/protein pore.

For (ii), the identity of the polynucleotide may be measured in a variety of ways, and the identity of the polynucleotide may be measured in combination with or without measurement of the polynucleotide sequence. The former is simpler, and the polynucleotide is sequenced and thus identified. The latter may be done in several different ways. For example, the presence of a particular motif in the polynucleotide may be measured (without measuring the remaining sequence of the polynucleotide). Alternatively, the measurement of a particular electrical and/or optical signal in the method may identify that the polynucleotide is derived from a particular source.

For (iii), the sequence of the polynucleotide may be determined as previously described. Suitable sequencing methods, particularly those using electrical measurement methods, are described in Stoddart D et al., Proc Natl Acad Sci, 12; 106 (19) 7702-7, Lieberman KR et al., J Am Chem SoC., 2010; 132 (50) 17961-72, and International Application WO2000/28312.

For (iv), the secondary structure may be measured using a variety of methods. For example, if the method involves an electrical measurement method, a change in dwell time or a change in current flowing through the pore may be used to measure the secondary structure. This allows regions of single-stranded and double-stranded polynucleotides to be distinguished.

For (v), the presence or absence of any modification may be measured. The method preferably comprises determining whether the polynucleotide is modified by methylation, by oxidation, by damage, with one or more proteins, with one or more labels or tags, or by the absence of bases or nucleobases and sugars. Particular modifications will result in specific interactions with the pore, which may be measured using the methods described below. For example, methylcytosine may be distinguished from cytosine based on the current flowing through the pore during its interaction with each nucleotide.

The target polynucleotide is contacted with a mutant of a protein monomer/protein pore, for example, a mutant of a protein monomer/protein pore as in the embodiments. The mutant of the protein monomer/protein pore is generally present in a membrane. Suitable membranes are as previously described. The method may be performed using any device suitable for studying a system of the membrane/protein pore or mutant of the porin monomer, in which the mutant of the protein monomer/protein pore is present in the membrane. The method may be performed using any device suitable for use in transmembrane pore sensing. For example, the device comprises a chamber containing an aqueous solution and a barrier dividing the chamber into two parts. The barrier generally has a hole in which a membrane containing a pore is formed. Alternatively, the barrier forms a membrane in which a mutant of a protein monomer/protein pore is present. The method may be performed using the device described in International Application No. PCT/GB08/000562 (WO 2008/102120).

Various different types of measurements may be performed. This includes, but is not limited to, electrical measurements and optical measurements. The electrical measurements include voltage measurements, capacitance measurements, current measurements, impedance measurements, tunneling measurements (Ivanov AP et al., Nano Lett., 2011, Jan 12; 11 (I): 279-85) and FET measurements (International Application TO 2005/124888). The optical measurements may be combined with the electrical measurements (Soni GV et al., Rev Sci Instrum., 2010, Jan; 81 (1) 014301). The measurement may be a transmembrane current measurement, for example, a measurement of an ionic current flowing through the pore. In one embodiment, the electrical measurements or optical measurements may be performed using conventional electrical measurements or optical measurements.

The electrical measurements may be performed using standard single-channel recording apparatus as described in Stoddart D et al., Proc Natl Acad Sci, 12; 106 (19) 7702-7, Lieberman KR et al., J Am Chem SoC., 2010; 132 (50) 17961-72, and International Application WO 2000/28312. Alternatively, the electrical measurements may be performed using multichannel systems, for example, as described in International Application WO2009/077734 and International Application WO 2011/067559.

The method is preferably performed using a potential applied across the membrane. The applied potential may be a voltage potential. Alternatively, the applied potential may be a chemical potential. An example of the method is using a salt gradient across a membrane, such as an amphiphilic molecular layer. The salt gradient is disclosed in Holden et al., J Am Chem SoC., 2007, Jul 11; 129 (27): 8650-5. In some cases, the current flowing through a mutant of a protein monomer/protein pore when a polynucleotide moves relative to the mutant of the protein monomer/protein pore is used to estimate or determine the sequence of the polynucleotide. This is strand sequencing.

The method may comprise measuring the current flowing through the pore when the polynucleotide moves relative to the pore. Therefore, the apparatus used in the method may also comprise circuitry capable of applying a potential and measuring an electrical signal through the membrane and the pore. The method may be performed using a patch clamp or a voltage clamp,
and may comprise measuring the current flowing through the pore when the polynucleotide moves relative to the pore. Suitable conditions for measuring ion currents through transmembrane protein pores are known in the art and are disclosed in the embodiments. The method is generally performed with a voltage applied across the membrane and the pore. The voltage used is generally from +5 V to -5 V, for example, from +4 V to -4 V, from +3 V to -3 V, or from +2 V to -2 V. The voltage used is generally from -600 mV to +600 V or -400 mV to +400 mV. The voltage used is preferably in a range having a lower limit selected from -400 mV, -300 mV, -200 mV, -150 mV, -100 mV, -50 mV, -20 mV, and 0 mV, and an upper limit independently selected from +10 mV, +20 mV, +50 mV, +100 mV, +150 mV, +200 mV, +300 mV, and +400 mV. The voltage used is more preferably in the range of 100 mV to 240 mV and most preferably in the range of 120 mV to 220 mV. By using an increased applied potential, the identification of different nucleotides by a pore may be enhanced.

The method is generally performed in the presence of any charge carrier, for example, a metal salt such as an alkali metal salt, a halide salt such as a chloride salt, for example, an alkali metal chloride salt. The charge carriers may include an ionic liquid or an organic salt, such as tetramethylammonium chloride, trimethylphenylammonium chloride, phenyltrimethylammonium chloride, or 1-ethyl-3-methylimidazolium chloride. In the exemplary device described above, the salt is present in the aqueous solution in the chamber. Potassium chloride (KCl), sodium chloride (NaCl), cesium chloride (CsCl), or a mixture of potassium ferrocyanide and potassium ferricyanide is generally used. KCl, NaCl, and the mixture of potassium ferrocyanide and potassium ferricyanide are preferred. The charge carriers may be asymmetric on the membrane. For example, the type and/or concentration of the charge carriers may be different on each side of the membrane.

The concentration of the salt may be saturated. The concentration of the salt may be 3 M or less, and is generally 0.1 to 2.5 M, 0.3 to 1.9 M, 0.5 to 1.8 M, 0.7 to 1.7 M, 0.9 to 1.6 M, or 1 to 1.4 M. The concentration of the salt is preferably 150 mM to 1 M. The method is preferably performed using a salt concentration of at least 0.3 M, for example, at least 0.4 M, at least 0.5 M, at least 0.6 M, at least 0.8 M, at least 1.0 M, at least 1.5 M, at least 2.0 M, at least 2.5 M, or at least 3.0 M. High salt concentrations provide a high signal-to-noise ratio and allow the presence of a nucleotide to be identified in the background of normal current fluctuations to be indicated by the current.

The method is generally performed in the presence of a buffer. In the exemplary device described above, the buffer is present in the aqueous solution in the chamber. Any buffer may be used in the method of the present invention. Generally, the buffer is a phosphate buffer. Other suitable buffers are HEPES or Tris-HCl buffers. The method is generally performed at a pH of 4.0 to 12.0, 4.5 to 10.0, 5.0 to 9.0, 5.5 to 8.8, 6.0 to 8.7, 7.0 to 8.8, or 7.5 to 8.5. The pH value used is preferably about 7.5.

The method may be performed at a temperature of 0 °C to 100 °C, 15 °C to 95 °C, 16 °C to 90 °C, 17 °C to 85 °C, 18 °C to 80 °C, 19 °C to 70 °C, or 20 °C to 60 °C. The method is generally performed at room temperature. The method is optionally performed at a temperature that supports enzyme functions, for example, about 37 °C.

In one embodiment, the method for determining the presence, absence, or one or more characteristics of a target analyte (e.g., a polynucleotide) comprises coupling the target analyte to a membrane; and the target analyte interacting (e.g., contacting) with the protein pore present in the membrane, such that the target analyte moves relative to the protein pore (e.g., passes through the protein pore). In one embodiment, the current through the protein pore is measured when the target analyte moves relative to the protein pore, thereby determining the presence, absence, or one or more characteristics of the target analyte (e.g., the sequence of the polynucleotide).

### Polynucleotide binding protein

The characterization method of the embodiments preferably comprises contacting a polynucleotide with a polynucleotide binding protein, such that the protein controls the movement of the polynucleotide relative to, e.g., through, a mutant of a protein monomer/protein pore.

More preferably, the method comprises (a) contacting the polynucleotide with the mutant of the protein monomer/protein pore and the polynucleotide binding protein, such that the protein controls the movement of the polynucleotide relative to, e.g., through, the mutant of the protein monomer/protein pore, and (b) acquiring one or more measurements when the polynucleotide moves relative to the mutant of the protein monomer/protein pore, wherein the measurements are indicative of one or more characteristics of the polynucleotide, thereby characterizing the polynucleotide.

More preferably, the method comprises (a) contacting the polynucleotide with the mutant of the protein monomer/protein pore and the polynucleotide binding protein, such that the protein controls the movement of the polynucleotide relative to, e.g., through, the mutant of the protein monomer/protein pore, and (b) measuring a current through the mutant of the protein monomer/protein pore when the polynucleotide moves relative to the mutant of the protein monomer/protein pore, wherein the current is indicative of one or more characteristics of the polynucleotide, thereby characterizing the polynucleotide.

The polynucleotide binding protein may be any protein capable of binding a polynucleotide and controlling the movement thereof through a pore. The polynucleotide binding protein generally interacts with a polynucleotide and modifies at least one property of the polynucleotide. The protein may modify a polynucleotide by cleaving it to form individual nucleotides or short strands of nucleotides such as dinucleotides or trinucleotides. The protein may modify a polynucleotide by orienting it or moving it to a specific position, i.e., controlling its movement.

The polynucleotide binding protein is preferably derived from a polynucleotide handling enzyme. The polynucleotide handling enzyme is a polypeptide that is capable of interacting with a polynucleotide and modifying at least one property of the polynucleotide. The enzyme may modify a polynucleotide by cleaving it to form individual nucleotides or short strands of nucleotides such as dinucleotides or trinucleotides. The enzyme may modify a polynucleotide by orienting it or moving it to a specific position. The polynucleotide handling enzyme does not need to exhibit enzymatic activity as long as it is capable of binding to a polynucleotide and controlling its movement through a pore. For example, the enzyme may be modified to remove its enzymatic activity, or may be used under conditions that prevent it from acting as an enzyme.

The polynucleotide handling enzyme is preferably a polymerase, an exonuclease, a helicase, and a topoisomerase such as a gyrase. In one embodiment, the enzyme is preferably a helicase, such as Hel308Mbu, Hel308Csy, Hel308Tga, Hel308Mhu, Tral Eco, XPD Mbu, Dda, or variants thereof. Any helicase may be used in the embodiments.

In one embodiment, any number of helicases may be used. For example, 1, 2, 3, 4, 5, 6, 7, 8, 9, 10, or more helicases may be used. In some embodiments, different numbers of helicases may be used.

The method of the embodiments preferably comprises contacting a polynucleotide with two or more helicases. The two or more helicases are generally the same helicase. The two or more helicases may be different helicases.

The two or more helicases may be any combination of the helicases described above. The two or more helicases may be two or more Dda helicases. The two or more helicases may be one or more Dda helicases and one or more TrwC helicases. The two or more helicases may be different variants of the same helicase.

The two or more helicases are preferably linked to each other. The two or more helicases are more preferably covalently linked to each other. The helicases may be linked in any order and by using any method.

### Kit

The present invention further provides a kit for characterizing a target analyte (e.g., a target polynucleotide). The kit comprises a pore and components of a membrane in the embodiments. The membrane is preferably formed from the components. The pore is preferably present in the membrane. The kit may comprise the components of any of the membranes disclosed above (e.g., an amphiphilic layer or a triblock copolymer membrane). The kit may further comprise a polynucleotide binding protein. Any of the polynucleotide binding proteins discussed above may be used.

In one embodiment, the membrane is an amphiphilic layer, a solid layer, or a lipid bilayer.

The kit may further comprise one or more anchors for coupling the polynucleotide to the membrane.

The kit is preferably used for characterizing a double-stranded polynucleotide and preferably comprises a Y adapter and a hairpin loop adapter.

The Y adapter preferably has one or more helicases linked, and the hairpin loop adapter preferably has one or more molecular brakes linked. The Y adapter preferably comprises one or more first anchors for coupling the polynucleotide to the membrane, the hairpin loop adapter preferably comprises one or more second anchors for coupling the polynucleotide to the membrane, and the coupling strength of the hairpin loop adapter to the membrane is preferably greater than the coupling strength of the Y adapter to the membrane.

The kit may additionally comprise one or more other reagents or instruments that enable any of the embodiments mentioned above to be performed. Such reagents or instruments include one or more of the following: suitable buffers (aqueous solutions), a device for obtaining a sample from an individual (such as a vessel or instrument containing a needle), a device for amplifying and/or expressing a polynucleotide, or voltage or patch clamp apparatus. The reagents may be present in the kit in a dry form, such that a fluid sample resuspends the reagents. Optionally, the kit may further comprise instructions to enable the kit to be used with the method of the present invention or details as to what organism the method may be used in.

### Apparatus (or device)

The present invention further provides an apparatus for characterizing a target analyte (e.g., a target polynucleotide). The apparatus comprises single or multiple mutants of a protein monomer/protein pores, and single or multiple membranes. The mutant of the protein monomer/protein pore is preferably present in the membrane. The number of pores and membranes is preferably equal. Preferably, a single pore is present in each membrane.

Preferably, the apparatus further comprises instructions for implementing the method of the embodiments. The apparatus may be any conventional apparatus for analyte analysis, for example, an array or chip. Any of the embodiments discussed in combination with the method of the embodiments is equally applicable to the apparatus. The apparatus may further comprise any of the characteristics present in the kit described herein. The apparatus used in the embodiments may specifically be a gene sequencer, QNome-9604, from QitanTech.

The above-mentioned prior art is incorporated herein by reference in its entirety.

The following examples are intended to illustrate the present invention without limiting it.

### Example 1

In the example, a wild-type porin was derived from Thermodesulfovibrio sp. N1, the amino acid sequence of the wild-type porin was set forth in SEQ ID NO: 1, and the nucleotide sequence encoding this amino acid sequence was set forth in SEQ ID NO: 2. Mutant 1 of a porin monomer was a wild-type porin having mutations at positions 74-81 corresponding to SEQ ID NO: 1; specifically, APGNATNF at positions 74-81 were substituted with PYPANA. A protein pore comprising the mutant 1 of the porin monomer was mutant pore 1. The amino acid sequence of the mutant 1 of the protein monomer was set forth in SEQ ID NO: 24, and the nucleic acid sequence was set forth in SEQ ID NO: 25.

### Example 2

In the example, a wild-type porin was derived from Thermodesulfovibrio sp. N1, the amino acid sequence of the wild-type porin was set forth in SEQ ID NO: 1, and the nucleotide sequence encoding this amino acid sequence was set forth in SEQ ID NO: 2. Mutant 2 of a porin monomer was a wild-type porin having mutations at positions 74-83 corresponding to SEQ ID NO: 1; specifically, APGNATNFST at positions 74-83 were substituted with AHPAATSP. A protein pore comprising the mutant 2 of the porin monomer was mutant pore 2. The amino acid sequence of the mutant 2 of the protein monomer was set forth in SEQ ID NO: 26.

### Example 3

In the example, a wild-type porin was derived from Thermodesulfovibrio sp. N1, the amino acid sequence of the wild-type porin was set forth in SEQ ID NO: 1, and the nucleotide sequence encoding this amino acid sequence was set forth in SEQ ID NO: 2. Mutant 3 of a porin monomer was a wild-type porin having mutations at positions 74-83 corresponding to SEQ ID NO: 1; specifically, APGNATNFST at positions 74-83 were substituted with PAASSLSP. A protein pore comprising the mutant 3 of the porin monomer was mutant pore 3. The amino acid sequence of the mutant 3 of the protein monomer was set forth in SEQ ID NO: 27.

### Example 4

In the example, a wild-type porin was derived from Thermodesulfovibrio sp. N1, the amino acid sequence of the wild-type porin was set forth in SEQ ID NO: 1, and the nucleotide sequence encoding this amino acid sequence was set forth in SEQ ID NO: 2. Mutant 4 of a porin monomer was a wild-type porin having mutations at positions 74-83 corresponding to SEQ ID NO: 1; specifically, APGNATNFST at positions 74-83 were substituted with PYPANASP. A protein pore comprising the mutant 4 of the porin monomer was mutant pore 4. The amino acid sequence of the mutant 4 of the protein monomer was set forth in SEQ ID NO: 28.

### Example 5

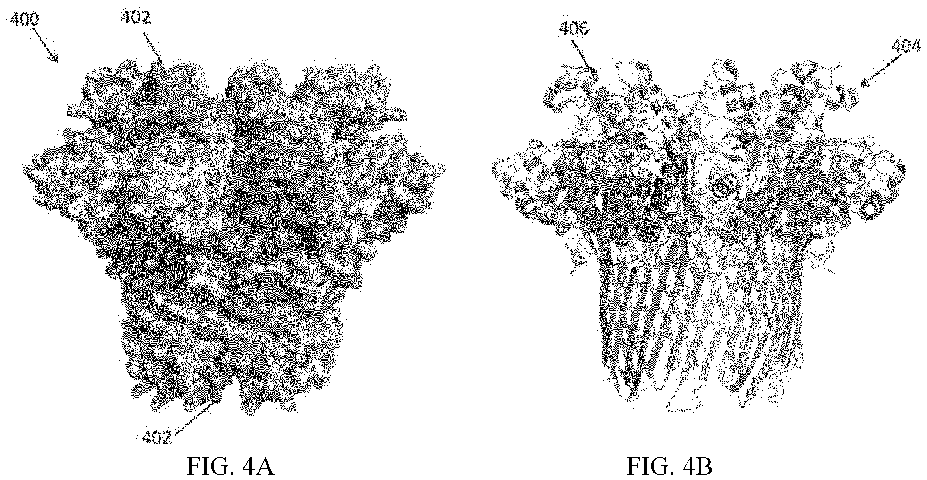

The wild-type porin was subjected to homologous modeling by adopting SWISS MODEL, and the amino acid of the wild-type porin monomer was set forth in SEQ ID NO: 1. FIG. 4A is a surface diagram 400 of a predicted protein structure, in which the darker portion shows a protein monomer 402. FIG. 4B is a ribbon model diagram 404, in which the darker portion shows a protein monomer 406.

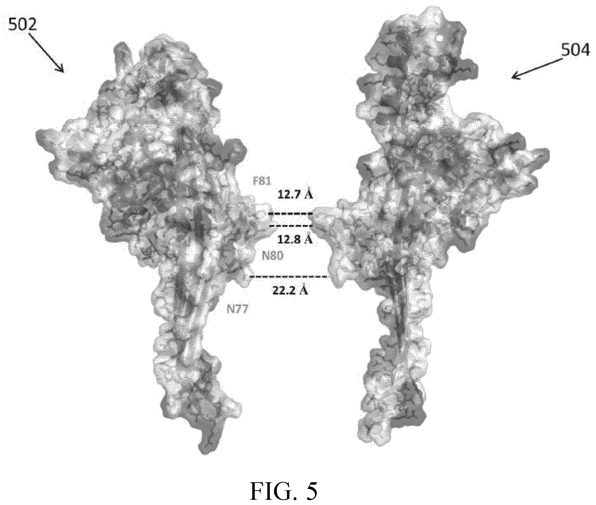

FIG. 5 shows a surface potential diagram of two protein monomers 502 and 504, in which the color depth indicates the electrical strength. The middle portion shows the amino acid composition of the constriction zone structure and the pore channel diameter of the constriction zone. The maximum diameter is 22.2 Å, followed by 12.8 Å, and the minimum diameter is 12.7 Å.

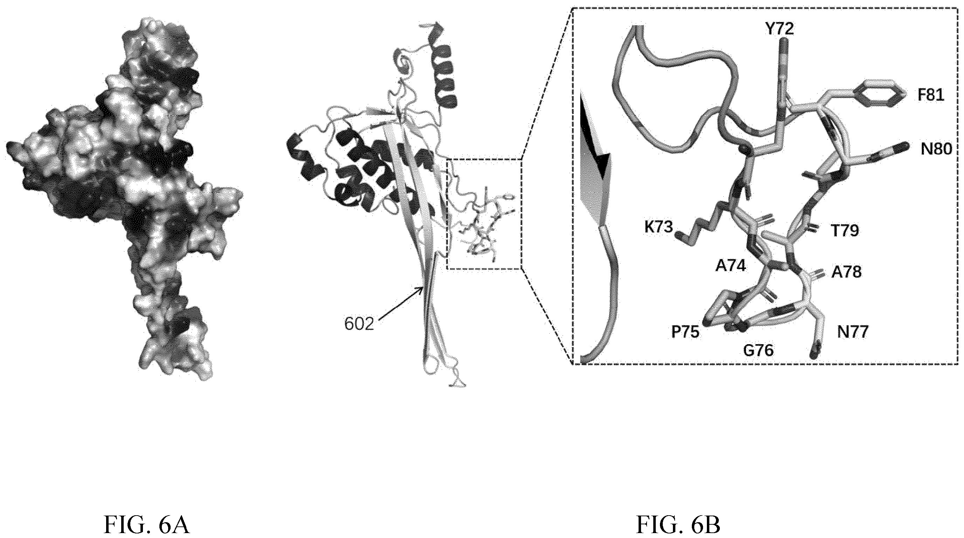

FIG. 6A shows a surface potential diagram of a wild-type protein monomer; FIG. 6B shows constituent elements of a secondary structure of the monomer and a stick model of a constriction zone of the monomer, and the enlarged portion shows the loop amino acid composition and numbering in the constriction zone, wherein portion 602 was an amino acid residue pointing to a central region of a protein pore channel.

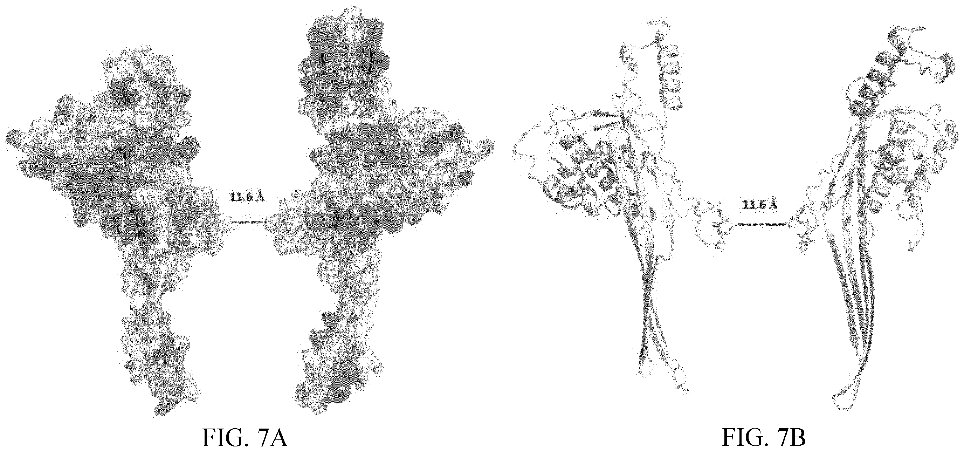

The mutant pore 1 was subjected to homologous modeling by adopting SWISS MODEL. FIG. 7A shows amino acid residue distribution characteristics in the constriction zone and constriction zone diameters of the mutant pore 1 (wild-type amino acid residues APGNATNF at positions 74-81 were substituted with PYPANA). FIG. 7A is a surface potential diagram, mainly showing the electrical characteristics of the surfaces and the reading head regions of the opposite monomers of the porin. The surface charge characteristics of the porin were closely related to the improvement of through-pore efficiency by nucleic acid recruitment, and the electrical characteristics near the reading head were closely related to the fact whether the nucleic acid could effectively pass through the constriction zone of the pore channel so as to achieve sequencing. FIG. 7B is a ribbon diagram showing the amino acid composition near the reading heads of the opposite monomers and a constriction zone diameter thereof and showing the key amino acid residue distribution of the narrow region of the protein pore channel of the mutant 1. The thickness of the constriction zone was reduced by the mutant structure, and the amino acid residue pointing to the center of the protein pore channel was asparagine at position 78. Hydrogen bonding interactions formed by the amino acid residues at positions 72-85 were closely related to the proper assembly of the protein pore channel complex. The predicted diameter of the narrowest region is about 11.6 Å.

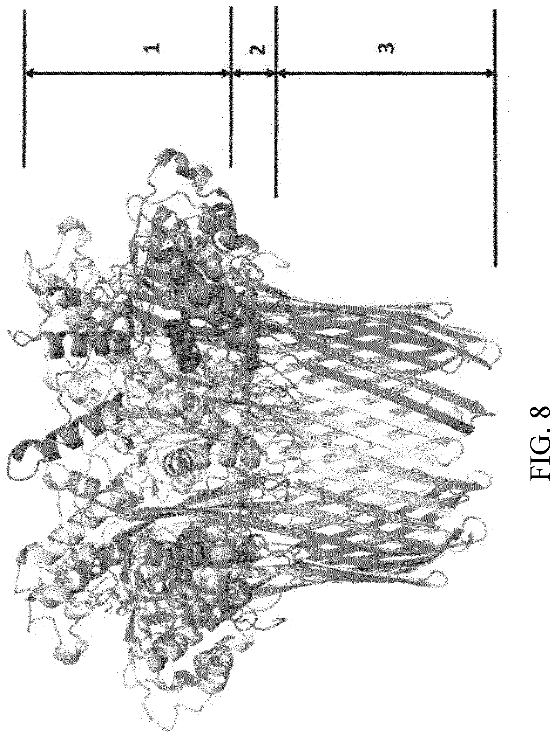

FIG. 8 shows the overall structural features of the mutant pore 1 based on homologous modeling, with region 1 corresponding to a corona formation region, region 2 corresponding to a constriction and loop region or a channel neck region, and region 3 corresponding to a transmembrane β-barrel region.

### Example 6. Preparation of DNA Constructs

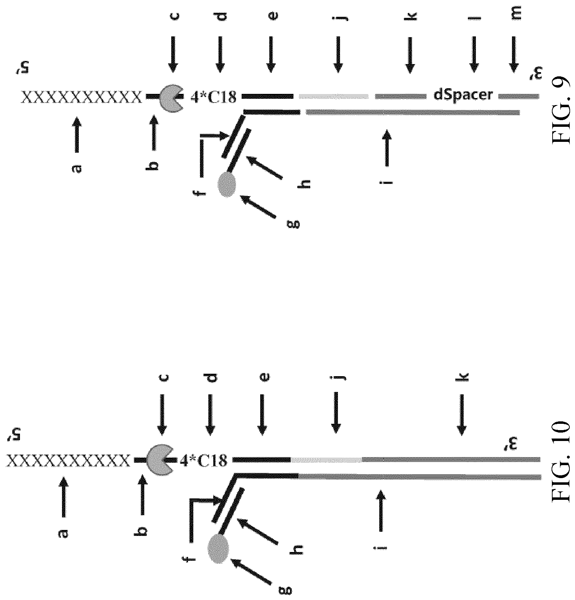

Two DNA constructs, BS7-4C3-SE1 and BS7-4C3-PLT, were prepared. The structure of BS7-4C3-SE1 is shown in FIG. 9, and the sequence information is shown below:
a: 30*C3
b: 5'-TTTTT TTTTT-3' (i.e., SEQ ID NO: 3)
c: rate-controlling protein
d: 4*C18
e: 5'-AATGT ACTTC GTTCA GTTAC GTATT GCT-3' (i.e., SEQ ID NO: 4)
f: 5'P-GC AATAC GTAAC TGAAC GAAGT TCACTATCGCATTCTCATGA-3' (i.e., SEQ ID NO: 5)
g: cholesterol tag
h: 5'- TCATG AGAAT GCGAT AGTGA -3' (i.e., SEQ ID NO: 6)
i: 5'-AAAAA AAAAA AAAAA AAAAA AAAAA AAAAA AAAAA AAAAA AAGCA ATACG TAACT GAACG AAGTA CATTA AAAAA AAAAA AAAAA AAAA-3' (i.e., SEQ ID NO: 7)
j: 5'-ATCCT TTTTT TTTTT TTTTT TTTT-3' (i.e., SEQ ID NO: 8)
k: 5'-AATGT ACTTC GTTCA GTTAC GTATT GCTTT TTTTT TTTTT TTTTT TTT-3' (i.e., SEQ ID NO: 9)
l: dSpacer
m: 5'-TTTTT TTTTT TTTTT TTTTT-3' (i.e., SEQ ID NO: 10)

The structure of BS7-4C3-PLT is shown in FIG. 10, and the sequence information is shown below:
a: 30*C3
b: 5'-TTTTT TTTTT-3' (i.e., SEQ ID NO: 11)
c: rate-controlling protein
d: 4*C18
e: 5'-AATGT ACTTC GTTCA GTTAC GTATT GCT-3' (i.e., SEQ ID NO: 12)
f: 5'P-GC AATAC GTAAC TGAAC GAAGT TCACTATCGCATTCTCATGA-3' (i.e., SEQ ID NO: 13)
g: cholesterol tag
h: 5'- TCATG AGAAT GCGAT AGTGA -3' (i.e., SEQ ID NO: 14)
i: 5'-AAAAAAAAAAAAAAAAAAAAAAAAAAAA (i.e., SEQ ID NO: 15)/dSpacer/ AAAAAAAAAAAA (i.e., SEQ ID NO: 16)/dSpacer/ AAAAAAAAAAAAAATCTCTGAATCTCTGAATCTCTGAATCTCTAAA AAAAAAAAAGAAAAAAAAAAAACAAAAAAAAAAAATAAAAAAAA AAAAAGCAATACGTAACTGAACGAAGTACATTAAAAAAAAAA (i.e., SEQ ID NO: 17)-3'
j: 5'-ATCCTTTTTTTTTTAATGTACTTCGTTCAGTTACGTATTGCT -3' (i.e., SEQ ID NO: 18)
k:
   5'P-TTTTTTTTTTTTATTTTTTTTTTTTGTTTTTTTTTTTTCTTTTTTTTT TTTAGAGATTCAGAGATTCAGAGATTCAGAGATTTTTTTTTTTTTT (i.e., SEQ ID NO: 19)/dSpacer/ TTTTTTTTTTTT (i.e., SEQ ID NO: 20)/iSpC3/ TTTTTTTTTTTTTTTTTTTTTTTTTTTT (i.e., SEQ ID NO: 21)-3'
   C3, C18, dSpacer, and iSpC3 were sequences of markers introduced to indicate the resolution characteristics of pore sequencing.

In this example, the rate-controlling protein c in FIGs. 9 and 10 is helicase Mph-MP1-E105C/A362C (having mutations E105C/A362C), the amino acid sequence is set forth in SEQ ID NO: 22, and the nucleic acid sequence is set forth in SEQ ID NO: 23.

### Example 7

The mutant pore 1 was used as a protein pore and detected by adopting a single-pore sequencing technique. After the insertion of a single porin with the amino acid sequence of the mutant 1 into a phospholipid bilayer, a buffer (625 mM KCl, 10 mM HEPES at pH of 8.0, and 50 mM MgCl₂) flowed through the system to remove any excess nanopores of the mutant 1. The DNA construct, BS7-4C3-SE1 or BS7-4C3-PLT (with a final concentration of 1-2 nM), was added to the nanopore experimental system of the mutant 1. After mixing well, the buffer (625 mM KCl, 10 mM HEPES at pH of 8.0, and 50 mM MgCl₂) flowed through the system to remove any excess DNA construct BS7-4C3-SE1 or BS7-4C3-PLT. A premix of the helicase (Mph-MP1-E105C/A362C with a final concentration of 15 nM) and fuel (ATP with a final concentration of 3 mM) was then added to the nanopore experimental system of the single mutant 1, and the sequencing of the mutant 1 porin was monitored at a voltage of +180 mV.

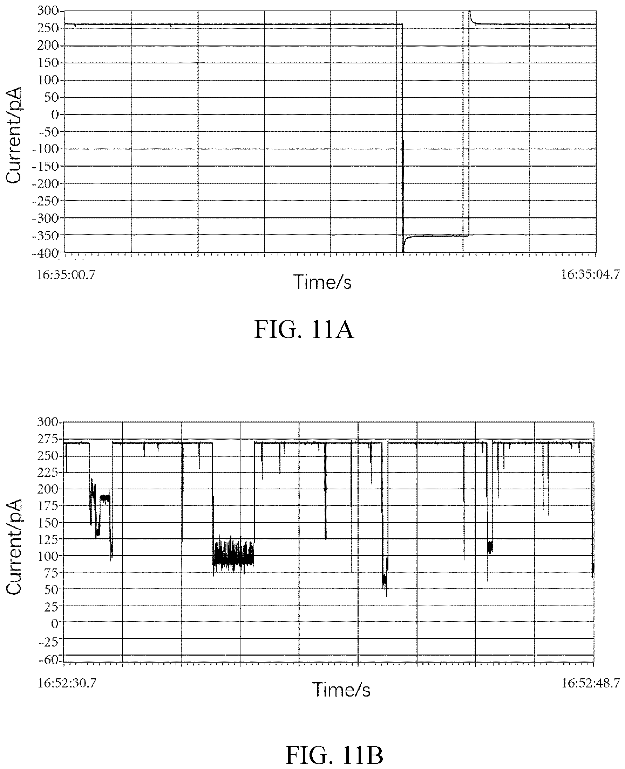

The mutant pore 1 was opened at a voltage of ±180 mV. FIG. 11A shows the application of a bias voltage to complete pore embedding in the system, wherein the upper and lower baselines indicate the current magnitudes for the positive and negative openings (about 270 pA and about -350 pA, respectively), and the pore channel was found with no gating mechanisms. FIG. 11B shows a scenario in which a nucleic acid passes through the pore of the mutant pore 1 at a voltage of +180 mV. After the addition of a single-stranded nucleic acid, the downward line shows a signal of the nucleic acid passing through the pore. This demonstrates that the mutant pore 1 can be stably opened and the nucleic acid can pass through the pore.

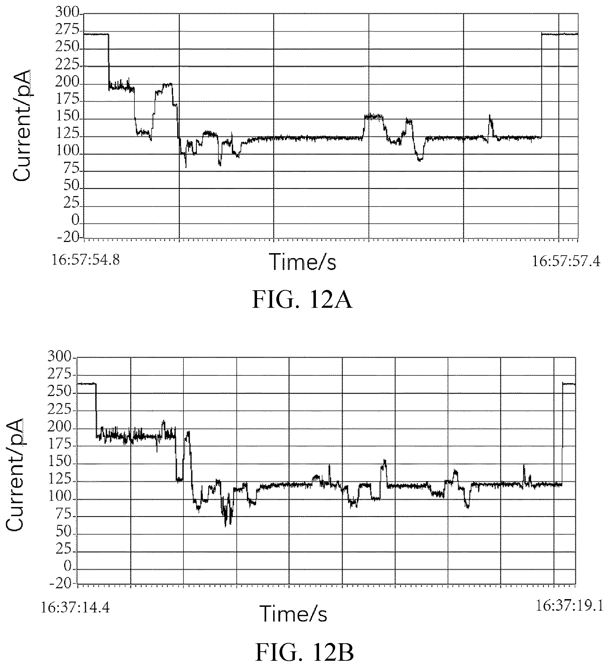

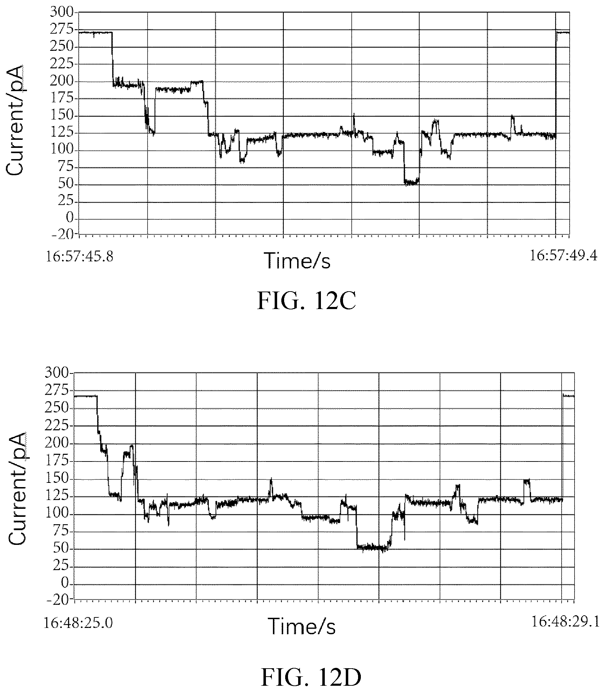

The single-pore sequencing technique was used to sequence the DNA construct BS7-4C3-SE1 through the mutant pore 1, and after the pore was embedded, the nucleic acid sequencing signal that appeared in the sequencing system was added. FIGs. 12A-12D show example current trajectories when the helicase Mph-MP1-E105C/A362C controls the translocation of the DNA construct BS7-4C3-SE1 through the mutant pore 1. According to the signal characteristics, the sequencing resolution, stability, signal consistency, and other related characteristics of the mutant pore 1 could be obtained. The pore had clear steps, significant jump distribution, and high-precision sequencing capability. From the characteristics of the four signals, the consistency of the sequencing signals was relatively high.

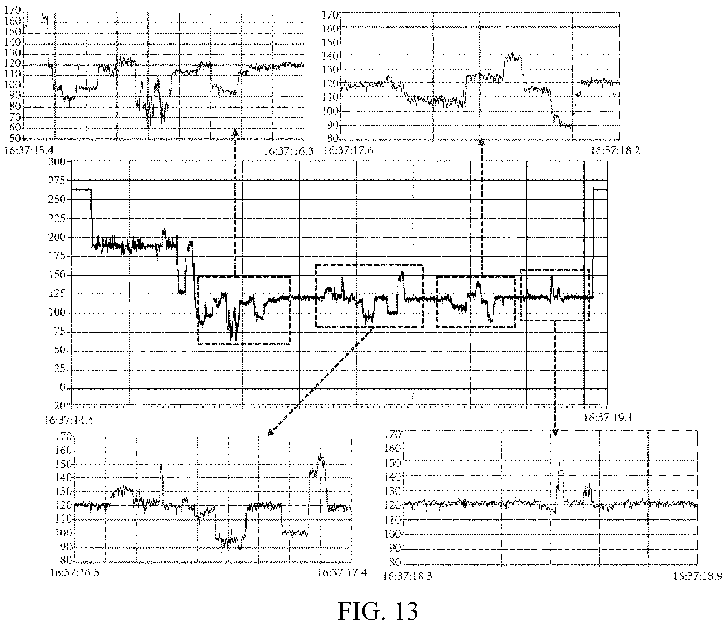

FIG. 13 is an enlarged result of the current trajectory shown in a portion of FIG. 12B. The diagram (middle diagram) with dashed boxes and arrows shows the result of filtering the original signal (for the two trajectories, the y-axis coordinate = current (pA), and the x-axis coordinate = time (s)). The dotted arrow indicative portions show enlarged results of the current trajectory. The enlarged region display of this single signal further demonstrates that the mutant pore had a high resolution for the nucleic acid sequencing.

### Example 8

Similar to Example 7, Example 8 used the mutant pore 2 for the empty test and through-pore test.

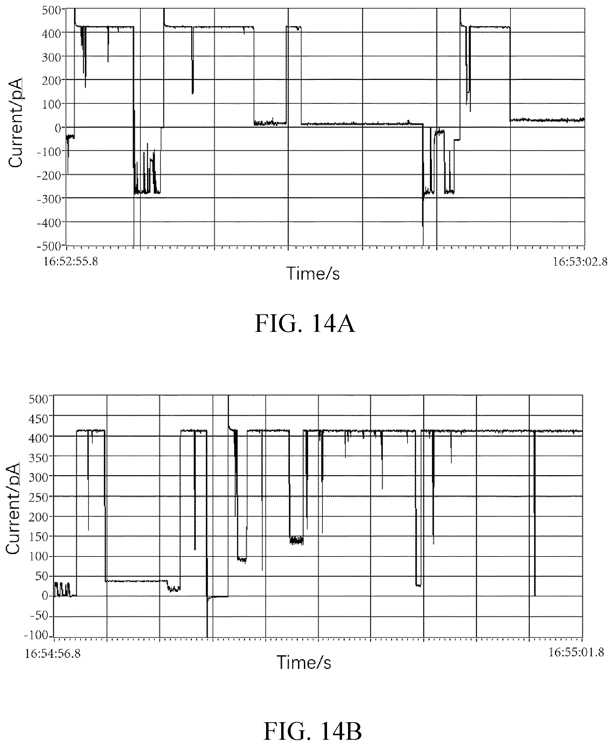

FIG. 14A shows an opening current and gated features of the mutant pore 2 at a voltage of ±180 mV. FIG. 14B shows a scenario in which a single-stranded nucleic acid passes through the pore of the mutant pore 2 at a voltage of +180 mV. The opening had certain instability, and gating was present in the reverse direction, so that the nucleic acid could pass through the pore. Compared with the mutant 1, the mutation of the core amino acid in the region is demonstrated to provide a remarkable effect on the signal characteristics and sequencing.

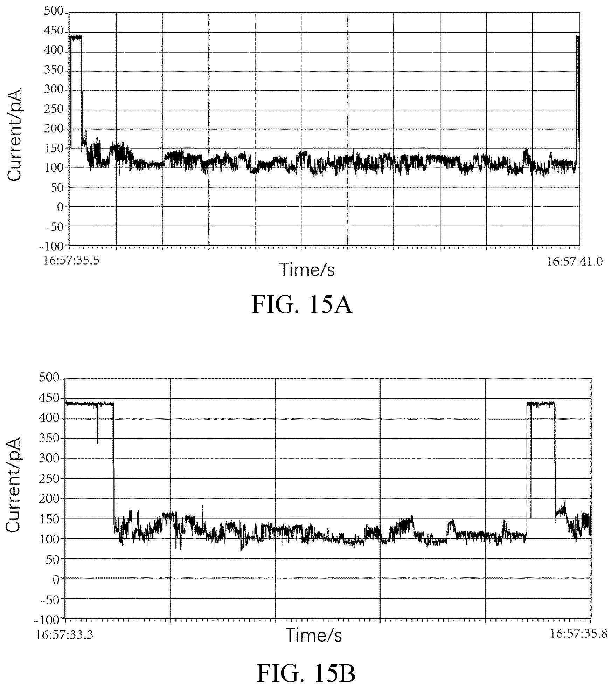

The single-pore sequencing technique was used to sequence the DNA construct BS7-4C3-PLT through the mutant pore 2, and after the pore was embedded, the nucleic acid sequencing signal that appeared in the sequencing system was added. FIGs. 15A and 15B show example current trajectories when the helicase Mph-MP1-E105C/A362C controls the translocation of the DNA construct BS7-4C3-PLT through the mutant pore 2. According to the signal characteristics, the mutant pore 1 has a higher resolution and lower noise than the mutant pore 2.

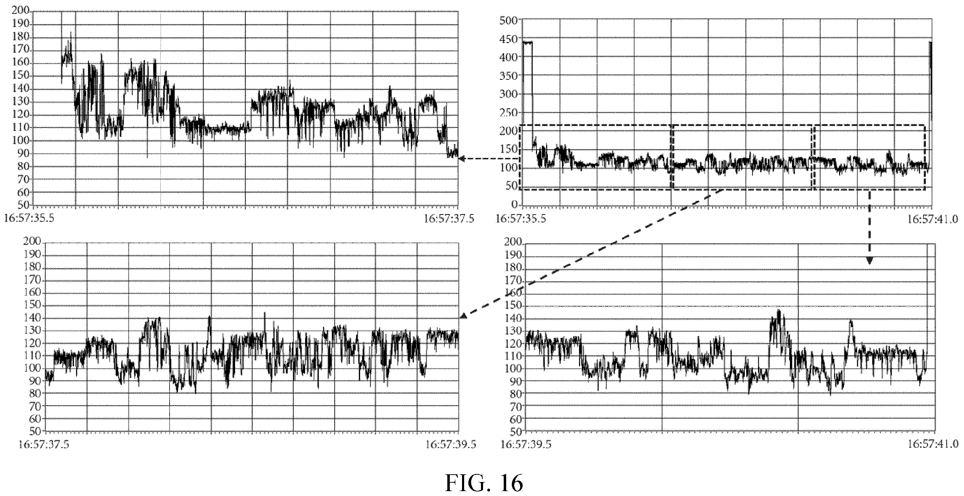

FIG. 16 is an enlarged result of the current trajectory shown in a portion of FIG. 15A. The diagram with dashed boxes and arrows shows the result of filtering the original signal (for the two trajectories, the y-axis coordinate = current (pA), and the x-axis coordinate = time (s)). The dotted arrow indicative portions show enlarged results of the current trajectory. The enlarged region display of this single signal indicates that the mutant pore 1 has a higher resolution than mutant pore 2.

The mutant pore 2 can also achieve sequencing, but the sequencing accuracy is worse than the mutant pore 1.

### Example 9

Similar to Example 7, Example 9 used the mutant pore 3 for the empty test and through-pore test.

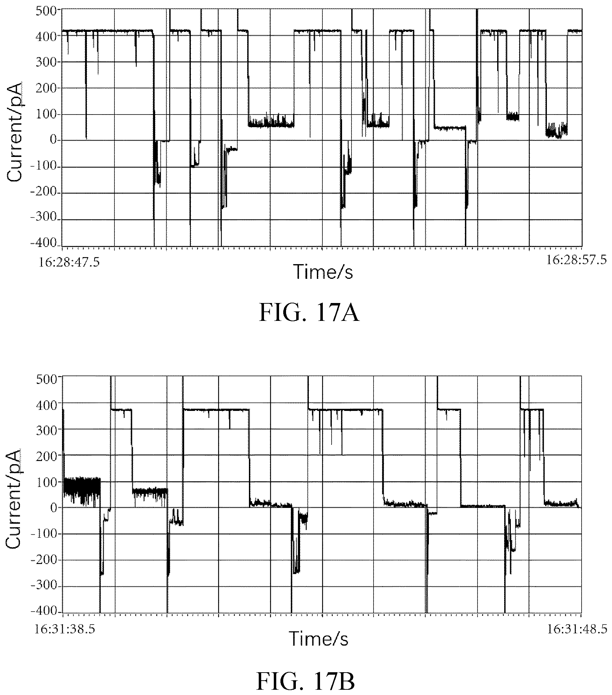

FIG. 17A shows an opening current and gated features of the mutant pore 3 at a voltage of ±180 mV. FIG. 17B shows a scenario in which a single-stranded nucleic acid passes through the pore of the mutant pore 3 at a voltage of +180 mV. The nucleic acid could pass through the pore. Compared with the mutant 1, the mutation of the core amino acid in the region is demonstrated to provide a remarkable effect on the signal characteristics and sequencing.

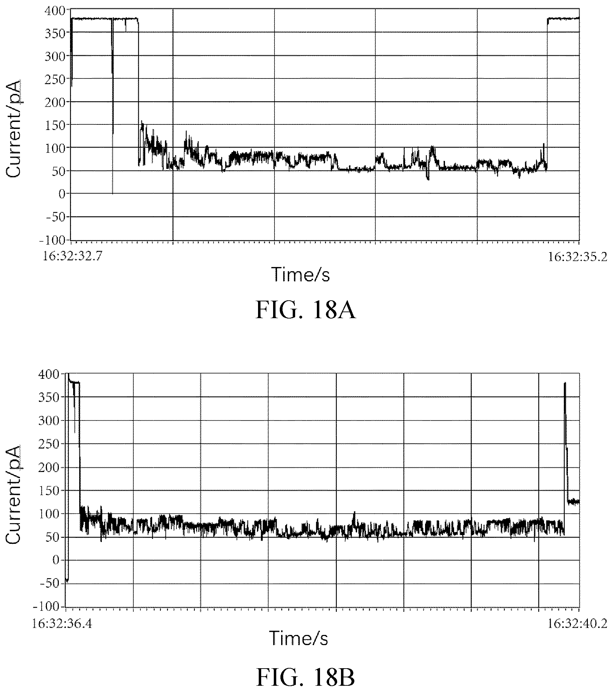

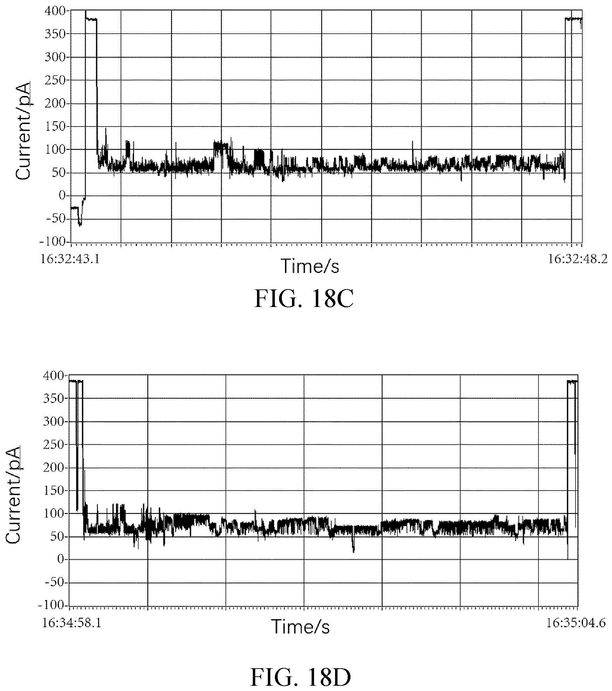

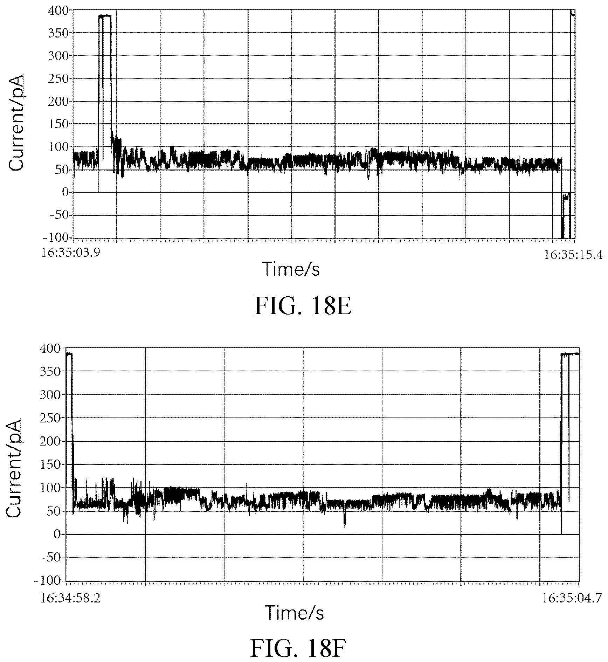

The single-pore sequencing technique was used to sequence the DNA construct BS7-4C3-PLT through the mutant pore 3, and after the pore was embedded, the nucleic acid sequencing signal that appeared in the sequencing system was added. FIGs. 18A-18F show example current trajectories when the helicase Mph-MP1-E105C/A362C controls the translocation of the DNA construct BS7-4C3-PLT through the mutant pore 3. According to the signal characteristics, the mutant pore 1 has a higher resolution and lower noise than the mutant pore 3.

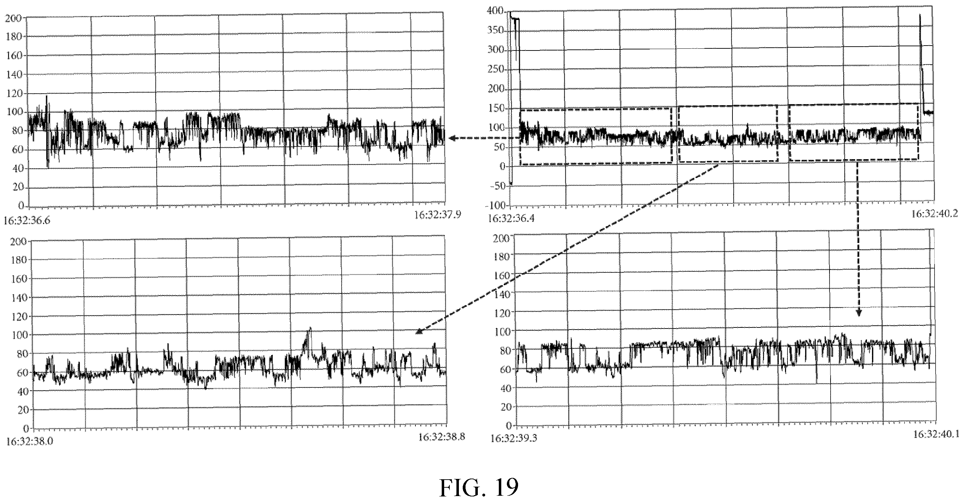

FIG. 19 is an enlarged result of the current trajectory shown in a portion of FIG. 18B. The diagram with dashed boxes and arrows shows the result of filtering the original signal (for the two trajectories, the y-axis coordinate = current (pA), and the x-axis coordinate = time (s)). The dotted arrow indicative portions show enlarged results of the current trajectory. The enlarged region display of this single signal further demonstrates that the mutant pore 1 has a higher resolution than the mutant pore 3.

The mutant pore 3 can also achieve sequencing, but the sequencing accuracy is worse than the mutant pore 1.

### Example 10

Similar to Example 7, Example 10 used the mutant pore 4 for the empty test and through-pore test.

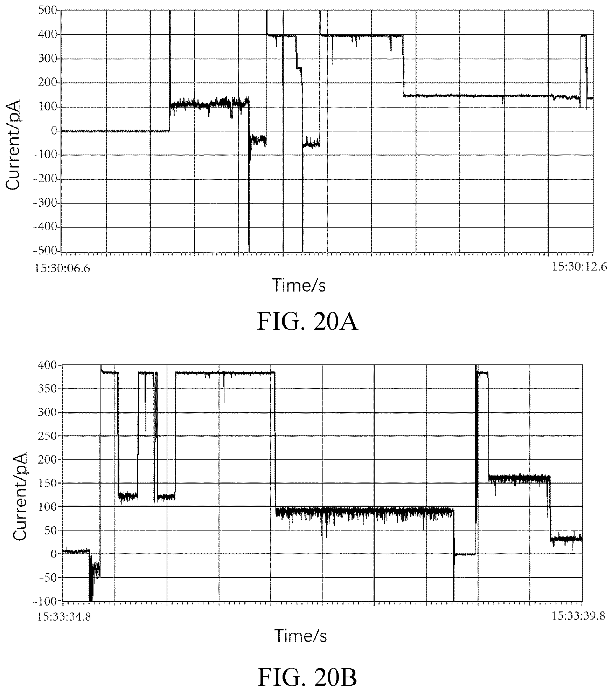

FIG. 20A shows an opening current and gated features of the mutant pore 4 at a voltage of ±180 mV. FIG. 20A shows a scenario in which a single-stranded nucleic acid passes through the pore of the mutant pore 4 at a voltage of +180 mV. The nucleic acid could pass through the pore. Compared with the mutant 1, the mutation of the core amino acid in the region is demonstrated to provide a remarkable effect on the signal characteristics and sequencing.

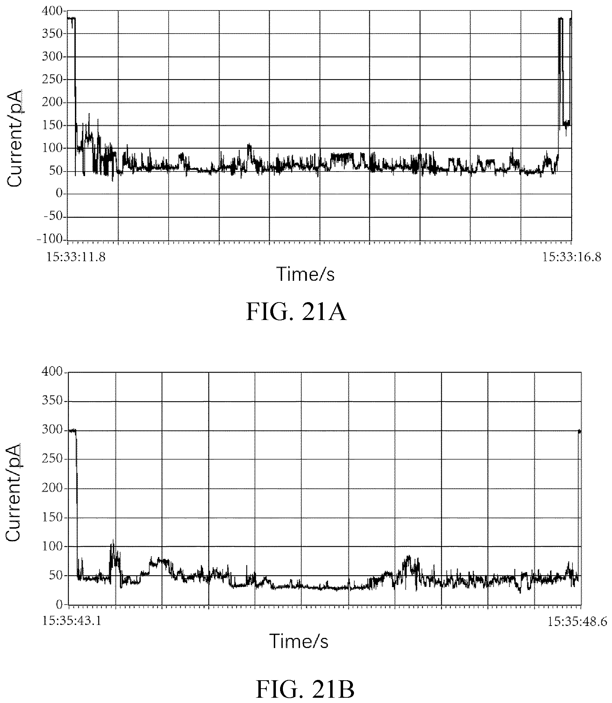

The single-pore sequencing technique was used to sequence the DNA construct BS7-4C3-PLT through the mutant pore 4, and after the pore was embedded, the nucleic acid sequencing signal that appeared in the sequencing system was added. FIGs. 21A-21F show example current trajectories when the helicase Mph-MP1-E105C/A362C controls the translocation of the DNA construct BS7-4C3-PLT through the mutant pore 4. According to the signal characteristics, the mutant pore 1 has a higher resolution and lower noise than the mutant pore 4.

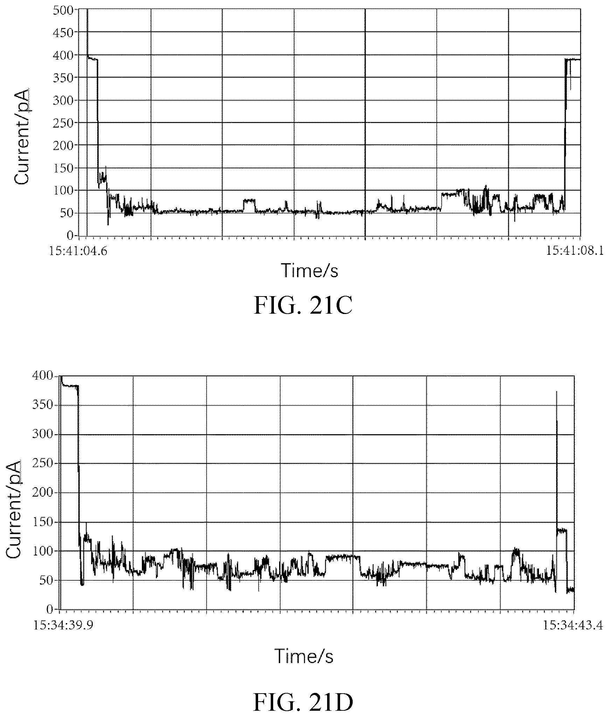

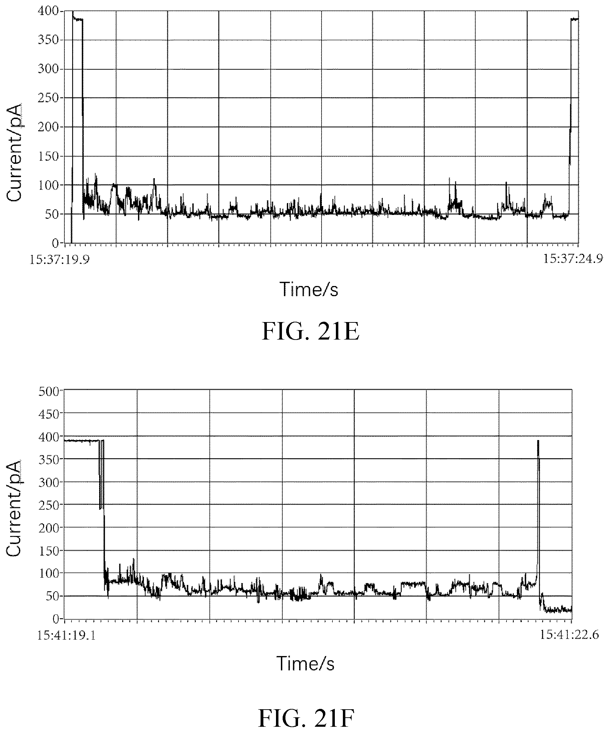

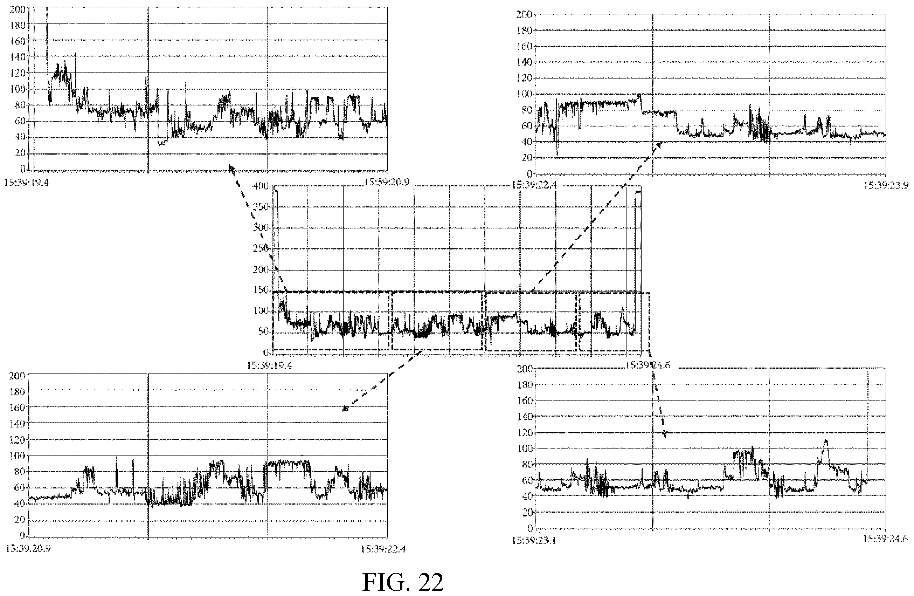

FIG. 22 is an enlarged result of the current trajectory shown in a portion of the example in FIG. 21. The diagram with dashed boxes and arrows shows the result of filtering the original signal (for the two trajectories, the y-axis coordinate = current (pA), and the x-axis coordinate = time (s)). The dotted arrow indicative portions show enlarged results of the current trajectory. The enlarged region display of this single signal indicates that the mutant pore 1 has a higher resolution than mutant pore 4.

The mutant pore 4 can also achieve sequencing, but the sequencing accuracy is worse than the mutant pore 1.

### Example 11

A recombinant plasmid containing the nucleic acid sequence of the mutant 1 of the porin monomer (SEQ ID NO: 25) was transformed into BL21(DE3) competent cells by heat shock, and 0.5 mL of LB culture medium was added. The cells were cultured at 30 °C for 1 h, and then a proper amount of bacterial liquid was taken and used to coated an ampicillin-resistant solid LB plate. The plate was cultured at 37 °C overnight. Monoclonal colonies were picked the next day and inoculated into 50 mL of liquid LB culture medium having ampicillin resistance, and the colonies were cultured at 37 °C overnight. The colonies were transferred to an ampicillin-resistant TB liquid culture medium at an inoculation amount of 1% for expansion culture. The colonies were cultured at 37 °C and 220 rpm and continuously measured for OD600 values. When OD600 = 2.0-2.2, the culture liquid in the TB culture medium was cooled to 16 °C, and isopropyl β-D-thiogalactoside (IPTG) was added to induce the expression, such that the final concentration reached 0.015 mM. After the expression was induced for 20-24 h, the bacteria were collected by centrifugation. The bacteria were resuspended in a crushing buffer and then crushed at high pressure. The mixed solution was purified by Ni-NTA affinity chromatography, and a target elution sample was collected. The mutants 2-4 of the porin monomer were purified and obtained according to the method described above.

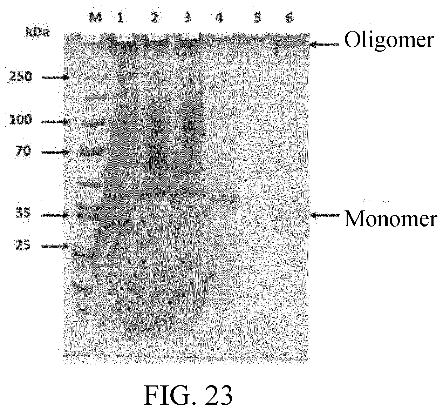

Illustratively, FIG. 23 shows protein purification results of the mutant 1, and SDS-PAGE electrophoresis results of the separated different components are shown in lanes 1-6.

## Claims

1. A mutant of a porin monomer, wherein an amino acid sequence of the mutant of the porin monomer comprises the sequence set forth in SEQ ID NO: 1 or a sequence having at least 99%, 98%, 97%, 96%, 95%, 90%, 80%, 70%, 60%, or 50% identity thereto, and wherein the amino acid sequence of the mutant of the porin monomer comprises mutations at one or more of positions corresponding to A74, P75, G76, N77, A78, T79, N80, and F81 of SEQ ID NO: 1.

2. The mutant of the porin monomer according to claim 1, wherein the amino acid sequence of the mutant of the porin monomer comprises:
(1) an insertion, a deletion, and/or a substitution of an amino acid at one or more of positions corresponding to A74, P75, G76, N77, A78, T79, N80, and F81 of SEQ ID NO: 1; or (2) an insertion, a deletion, and/or a substitution of an amino acid at one or more of positions corresponding to A74, P75, G76, N77, A78, T79, N80, F81, S82, and T83 of SEQ ID NO: 1.

3. The mutant of the porin monomer according to any one of the preceding claims, wherein the sequence set forth in SEQ ID NO: 1 is derived from Thermodesulfovibrio sp. N1.

4. The mutant of the porin monomer according to any one of the preceding claims, wherein the amino acid mutation of the mutant of the porin monomer is selected from the group consisting of:
(a) mutations from amino acids APGNATNF corresponding to positions 74-81 of SEQ ID NO: 1 to M₁M₂M₃M₄M₅M₆, wherein M₁ is selected from 0 to 1 of P; M₂ is selected from 0 to 3 of Y, F, and W; M₃ is selected from 0 to 1 of P; M₄ is selected from 0 to 5 of A, G, V, L, and I; M₅ is selected from 0 to 4 of N, D, E, and Q; M₆ is selected from 0 to 5 of A, G, V, L, and I;
(b) mutations from amino acids APGNATNFST corresponding to positions 74-83 of SEQ ID NO: 1 to M₇M₈M₉M₁₀M₁₁M₁₂ M₁₃M₁₄, wherein M₇ is selected from 0 to 5 of A, G, V, L, and I; M₈ is selected from 0 to 3 of H, K, and R; M₉ is selected from 0 to 1 of P; M₁₀ is selected from 0 to 5 of A, G, V, L, and I; M₁₁ is selected from 0 to 5 of A, G, V, L, and I; M₁₂ is selected from 0 to 5 of T, S, C, U, and M; M₁₃ is selected from 0 to 5 of S, C, U, T, and M; M₁₄ is selected from 0 to 1 of P;
(c) mutations from the amino acids APGNATNFST corresponding to positions 74-83 of SEQ ID NO: 1 to M₁₅M₁₆M₁₇M₁₈M₁₉M₂₀M₂₁M₂₂, wherein M₁₅ is selected from 0 to 1 of P; M₁₆ is selected from 0 to 5 of A, G, V, L, and I; M₁₇ is selected from 0 to 5 of A, G, V, L, and I; M₁₈ is selected from 0 to 5 of S, C, U, T, and M; M₁₉ is selected from 0 to 5 of S, C, U, T, and M; M₂₀ is selected from 0 to 5 of L, G, A, V, and I; M₂₁ is selected from 0 to 5 of S, C, U, T, and M; M₂₂ is selected from 0 to 1 of P; and
(d) mutations from the amino acids APGNATNFST corresponding to positions 74-83 of SEQ ID NO: 1 to M₂₃M₂₄M₂₅M₂₆M₂₇M₂₈M₂₉M₃₀, wherein M₂₃ is selected from 0 to 1 of P; M₂₄ is selected from 0 to 3 of Y, F, and W; M₂₅ is selected from 0 to 1 of P; M₂₆ is selected from 0 to 5 of A, G, V, L, and I; M₂₇ is selected from 0 to 4 of N, D, E, and Q; M₂₈ is selected from 0 to 5 of A, G, V, L, and I; M₂₉ is selected from 0 to 5 of S, C, U, T, and M; M₃₀ is selected from 0 to 1 of P.

5. The mutant of the porin monomer according to any one of the preceding claims, wherein the amino acid mutation of the mutant of the porin monomer is selected from the group consisting of:
(a) mutations from the amino acids APGNATNF corresponding to positions 74-81 of SEQ ID NO: 1 to PYPANA;
(b) mutations from the amino acids APGNATNFST corresponding to positions 74-83 of SEQ ID NO: 1 to AHPAATSP;
(c) mutations from the amino acids APGNATNFST corresponding to positions 74-83 of SEQ ID NO: 1 to PAASSLSP; and
(d) mutations from the amino acids APGNATNFST corresponding to positions 74-83 of SEQ ID NO: 1 to PYPANASP.

6. The mutant of the porin monomer according to any one of the preceding claims, wherein the mutant of the porin monomer comprises or consists of the amino acid sequence set forth in SEQ ID NO: 24, SEQ ID NO: 26, SEQ ID NO: 27, or SEQ ID NO: 28.

7. A protein pore comprising at least one mutant of the porin monomer according to any one of the preceding claims.

8. The protein pore according to claim 7, wherein the protein pore comprises at least two mutants of the porin monomer.

9. The protein pore according to any one of claims 7-8, wherein the channel diameter of the constriction zone of the protein pore is from 0.7 nm to 2.2 nm, from 0.9 nm to 1.6 nm, from 1.4 nm to 1.6 nm, or from 12.7 Å to 22.2 Å.

10. A complex for characterizing a target analyte, wherein the complex comprises the protein pore according to any one of claims 7-9 and a rate-controlling protein bound thereto.

11. A nucleic acid encoding the mutant of the porin monomer according to any one of claims 1-6, the protein pore according to any one of claims 7-9, or the complex according to claim 10.

12. The nucleic acid according to claim 11, wherein the nucleotide sequence of the porin monomer is the sequence set forth in SEQ ID NO: 2.

13. A vector or a genetically engineered host cell comprising the nucleic acid according to any one of claims 11-12.

14. Use of the mutant of the porin monomer according to any one of claims 1-6, the protein pore according to any one of claims 7-9, the complex according to claim 10, the nucleic acid according to any one of claims 11-12, or the vector or host cell according to claim 13 in the detection of the presence, absence, or one or more characteristics of a target analyte or in the preparation of a product for detecting the presence, absence, or one or more characteristics of a target analyte.

15. A method for producing a protein pore or a polypeptide thereof, comprising transforming the host cell according to claim 13 with the vector according to claim 13, and inducing the host cell to express the protein pore according to any one of claims 7-9 or the polypeptide thereof.

16. A method for determining the presence, absence, or one or more characteristics of a target analyte, comprising:
a. contacting the target analyte with the protein pore according to any one of claims 7-9, the complex according to claim 10, or the protein pore in the complex according to claim 10, such that the target analyte moves relative to the protein pore; and
b. acquiring one or more measurements when the target analyte moves relative to the protein pore, thereby determining the presence, absence, or one or more characteristics of the target analyte.

17. The method according to claim 16, wherein the method comprises:
the target analyte interacting with the protein pore present in a membrane, such that the target analyte moves relative to the protein pore.

18. A kit for determining the presence, absence, or one or more characteristics of a target analyte, comprising the mutant of the porin monomer according to any one of claims 1-6, the protein pore according to any one of claims 7-9, the complex according to claim 10, the nucleic acid according to any one of claims 11-12, or the vector or host cell according to claim 13, and a component of the membrane according to claim 17.

19. A device for determining the presence, absence, or one or more characteristics of a target analyte, comprising the protein pore according to any one of claims 7-9 or the complex according to claim 10, and the membrane according to claim 17.

20. The use, method, kit, or device according to any one of claims 14-19, wherein the target analyte comprises a polysaccharide, a metal ion, an inorganic salt, a polymer, an amino acid, a peptide, a protein, a nucleotide, an oligonucleotide, a polynucleotide, a dye, a drug, a diagnostic agent, an explosive, or an environmental contaminant;
preferably, the target analyte comprises a polynucleotide;
more preferably, the polynucleotide comprises DNA or RNA; and/or, the one or more characteristics are selected from (i) a length of the polynucleotide; (ii) an identity of the polynucleotide; (iii) a sequence of the polynucleotide; (iv) a secondary structure of the polynucleotide; and (v) whether the polynucleotide is modified; and/or, the rate-controlling protein in the complex comprises a polynucleotide binding protein.
